# EUROPEAN PATENT APPLICATION

(11) **EP 4 585 216 A1**
(43) Date of publication of application: **16.07.2025**
(21) Application number: 24151032.0
(22) Date of filing: 09.01.2024
(51) Int. Cl.: A61K 31/5415, A61P 1/16, C07D 279/02, A61P 35/00

(54) **HETEROCYCLIC COMPOUNDS FOR THE TREATMENT OF FATTY LIVER DISEASE**

(71) Applicant: ScandiEdge Therapeutics AB, 121 36 Johanneshov (SE)
(72) Inventor: MARDINOGLU, Adil, 412 60 Göteborg (SE); BORÉN, Jan, 414 67 Göteborg (SE); UHLÉN, Mathias, 181 90 Lidingö (SE); ISLAM, Zahidul, 34774 Istanbul (TR); IQBAL, Shazia, 34774 Istanbul (TR); ASHRAF, Sajda, 34774 Istanbul (TR)
(74) Representative: Kransell & Wennborg KB

(57) **Abstract**

There is provided a compound according to formula (I) 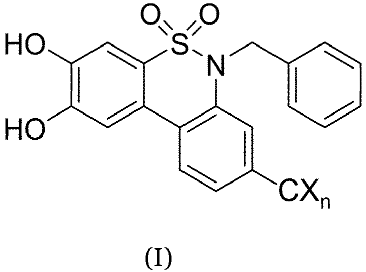 or a pharmaceutically acceptable salt or prodrug thereof wherein X is a halogen and n=1-3.

## Description

### TECHNICAL FIELD

The present disclosure relates to pharmaceutical compounds used for the treatment of fatty liver disease and hepatocellular carcinoma (HCC).

### BACKGROUND

Non-alcoholic fatty liver disease (NAFLD) refers to the accumulation of fat in liver independent of alcohol consumption [1,2]. NAFLD is the most common chronic liver disease in the Western world, which is associated with the development of cardiovascular diseases and type 2 diabetes [3]. The overall global prevalence of NAFLD is approximately 25%, and it is estimated to grow in the coming years [3,4]. Currently, there is no approved pharmacological therapy available to treat this disease. The existing therapies focus on weight loss (e.g., calorie restriction and exercise) and/or supplementation of vitamins e.g. vitamin E [5].

Pyruvate kinase (PK) is responsible for the final step in glycolysis, converting phosphoenolpyruvate (PEP) and adenosine diphosphate (ADP) to pyruvate (PYR) and adenosine triphosphate (ATP). PK is present in four different isoforms, PKM1, PKM2, PKR and PKL, each with multiple designations over time [8]. PKR is predominantly expressed in red blood cells, PKM1 is present in the skeletal muscle, brain and heart and PKM2 is expressed in proliferating cells, embryonic tissues and tumours [9,10]. The PKL is expressed exclusively in the liver but also in pancreatic β-cells, the small intestine, and the renal proximal tubule [11]. PKL -liver pyruvate kinase has been identified as a target to halt the progression of the NAFLD [2,6,7]. Hence, development of PKL specific inhibitors maybe extremely beneficial for treating NAFLD.

### SUMMARY

The present inventors have realized that there is a possibility to develop and optimize small molecules, which may be used in the treatment of fatty liver disease and hepatocellular carcinoma (HCC). The development/optimization was based on alteration of the chemical structure of Urolithin C in order to improve the efficacy of the small compounds in regard to PKL inhibition and reduction of triglyceride (TAG) levels.

Accordingly, there is provided a compound according to formula (I) 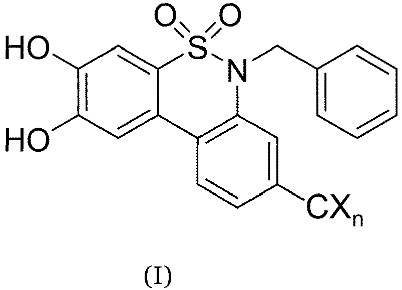 or a pharmaceutically acceptable salt or prodrug thereof wherein X is a halogen and n=1-3.

It has been shown that treating cells with a compound according to formula (I), reduces the expression levels of PKL as well as reduces the TAG content. A compound according to formula (I) has also been shown to penetrate into the cells which is crucial in order for the compound to be used in the treatment of NAFLD and HCC. Thus, a compound according to formula (I) is particularly well suited for use in pharmacological treatment of NAFLD.

### BRIEF DESCRIPTION OF THE DRAWINGS

Aspects and embodiments are now described, by way of example, with reference to the accompanying drawings, in which:

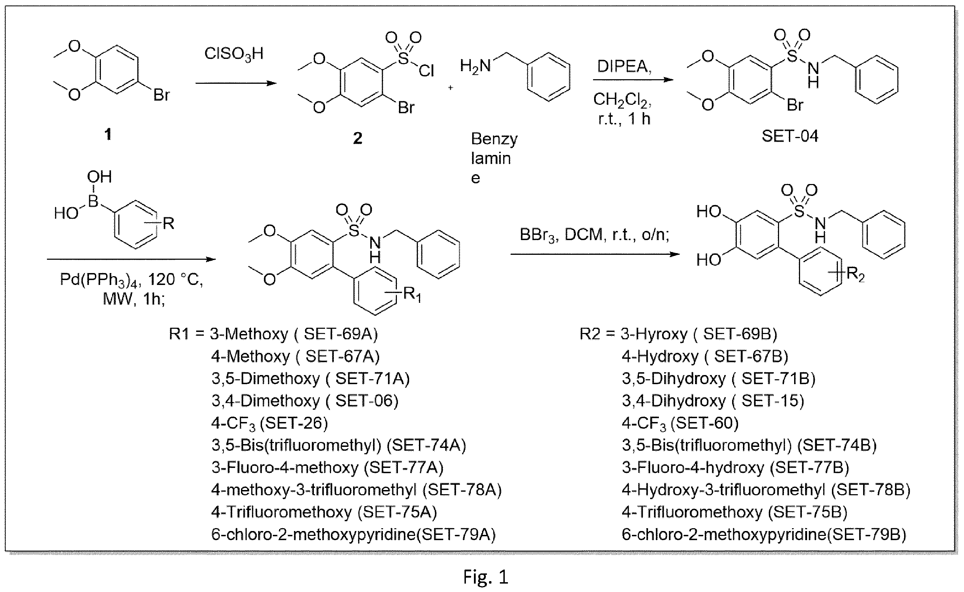

Fig 1 shows the synthesis of biaryl sulfonamides (with benzyl group).

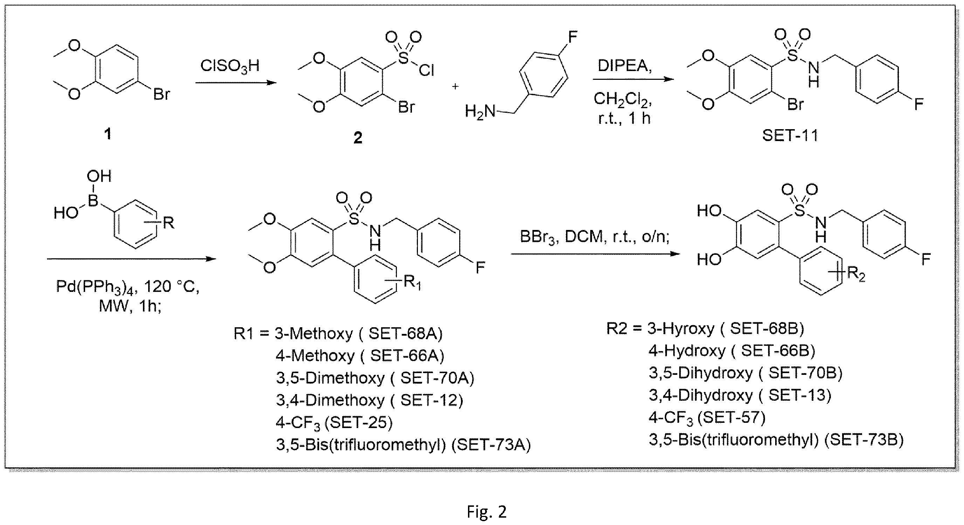

Fig 2 shows the synthesis of biaryl sulfonamides (with Fluorobenzyl group).

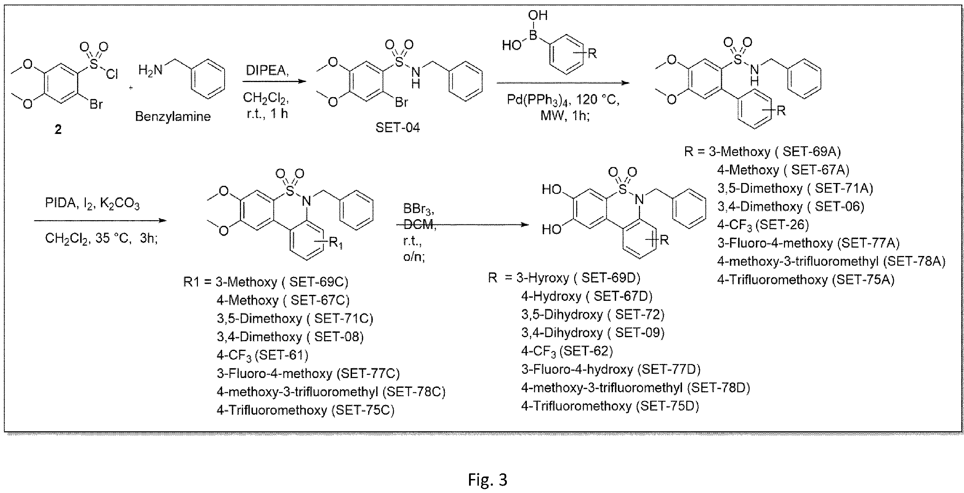

Fig 3 shows the synthesis of sultam derivatives of Urolithin C (with benzyl group).

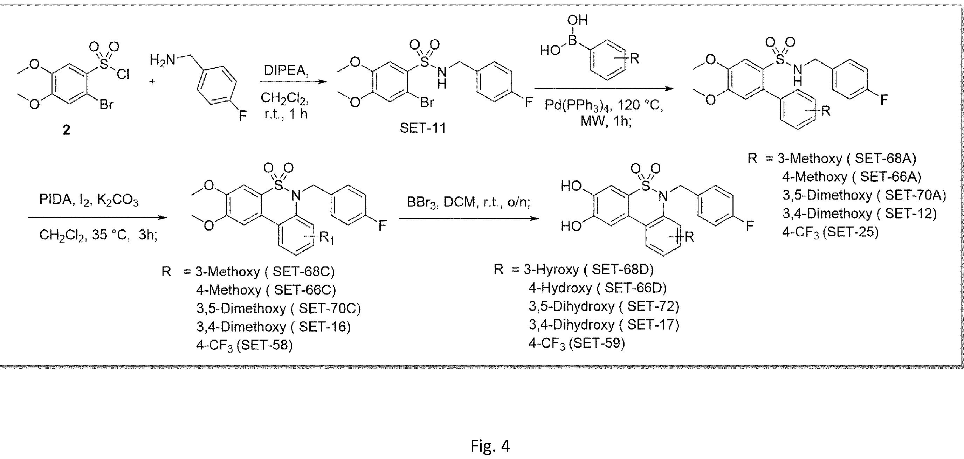

Fig 4 shows the synthesis of sultam derivatives of Urolithin C. (with Fluorobenzyl group).

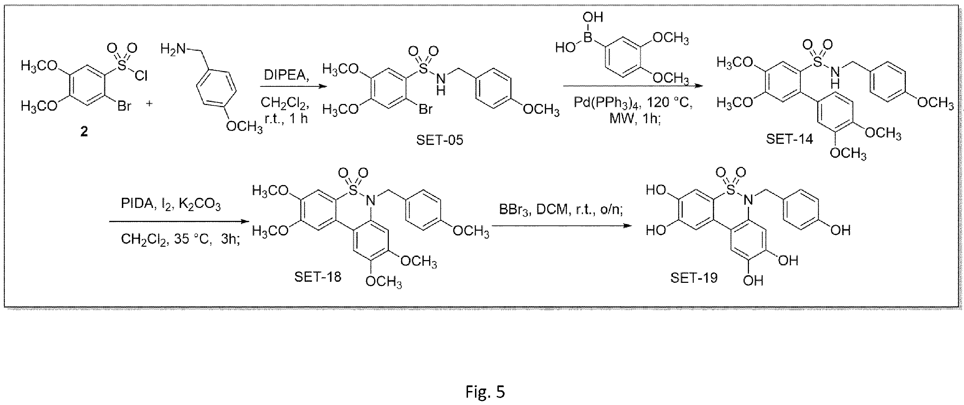

Fig 5 shows the synthesis of sultam derivatives of Urolithin C (with methoxybenzyl group).

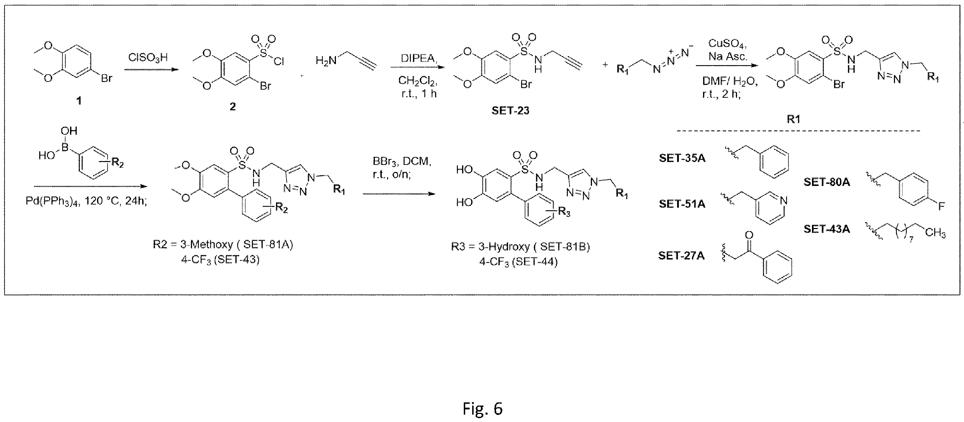

Fig 6 shows the synthesis of Biaryl Sulfonamide derivatives with different 1,2,3-Triazoles.

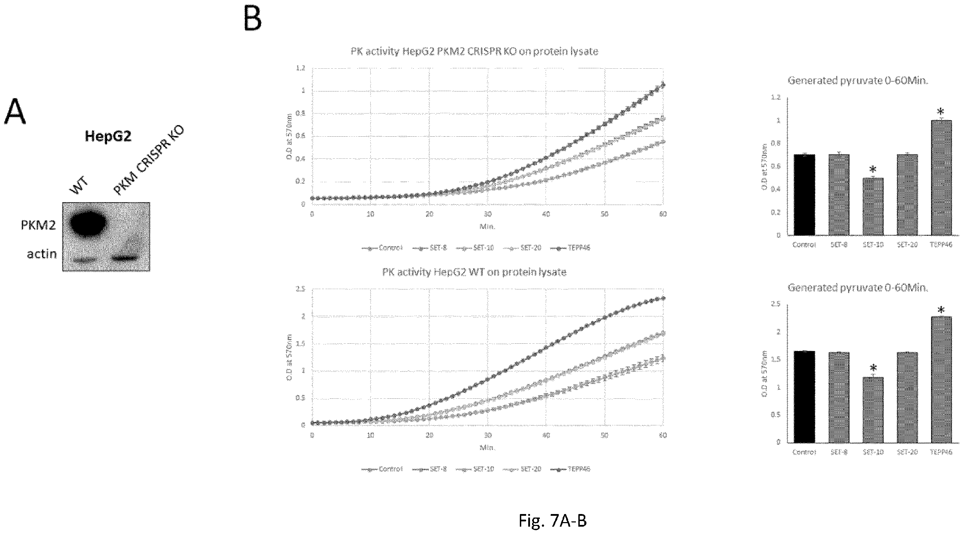

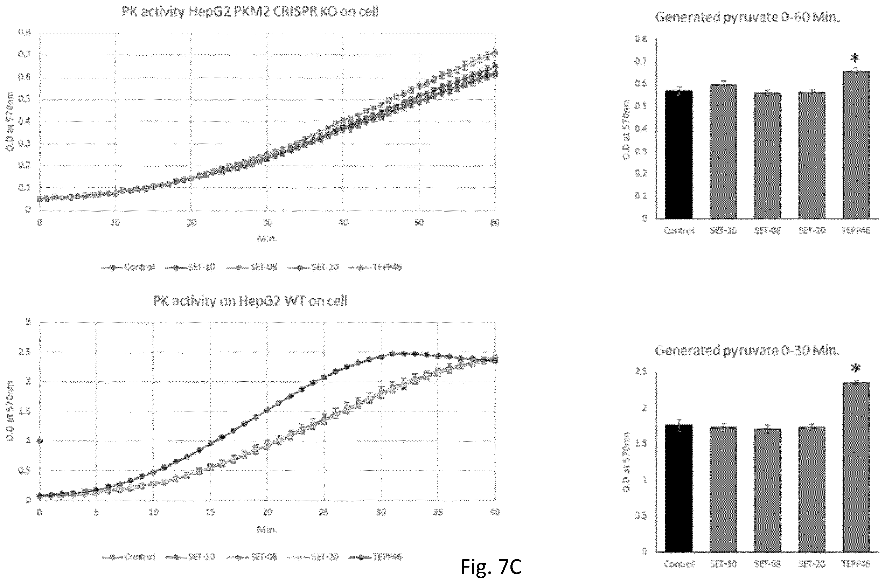

Fig 7 shows (A) the PKM2 expression of HepG2 WT cells and HepG2 CRISPR KO HepG2 WT cells. (B) PK activity in HepG2 WT and HepG2 PKM2 CRISPR KO protein lysate when treated with 10µM of SET-08, SET-10, SET-20 and TEPP46. Generated pyruvate (O.D value at 570nm) is shown with a histogram. Data are represented as mean ± SD, *p < 0.05, Student's t test. (C) PK activity in HepG2 WT and HepG2 PKM2 CRISPR KO cells when treated with 10 µM of SET-08, SET-10, SET-20 and TEPP46. Generated pyruvate (O.D value at 570nm) is shown with a histogram. Data are represented as mean ± SD, *p < 0.05, Student's t test.

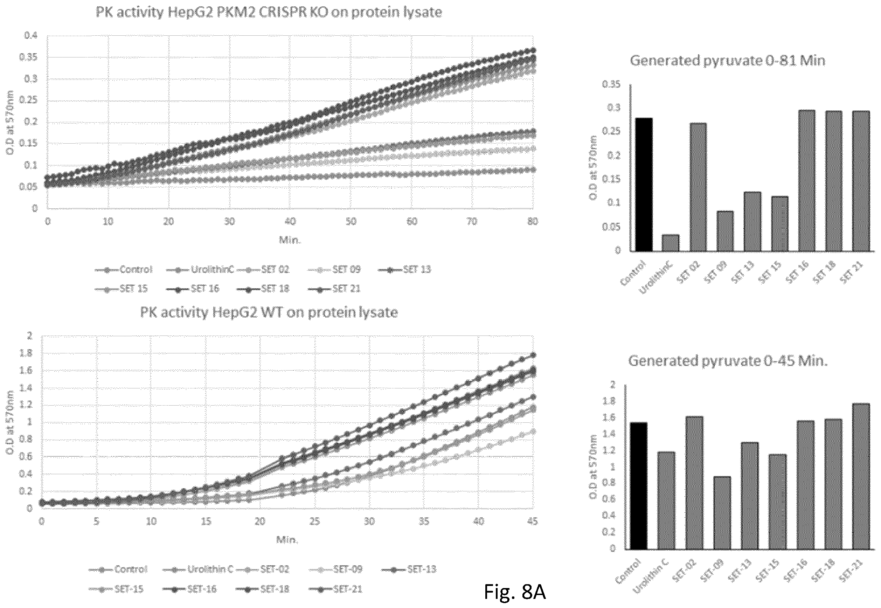

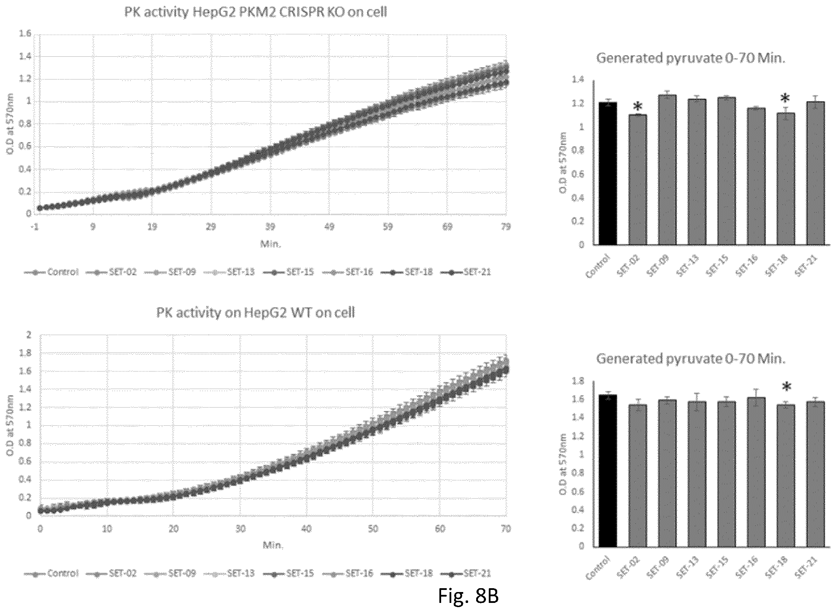

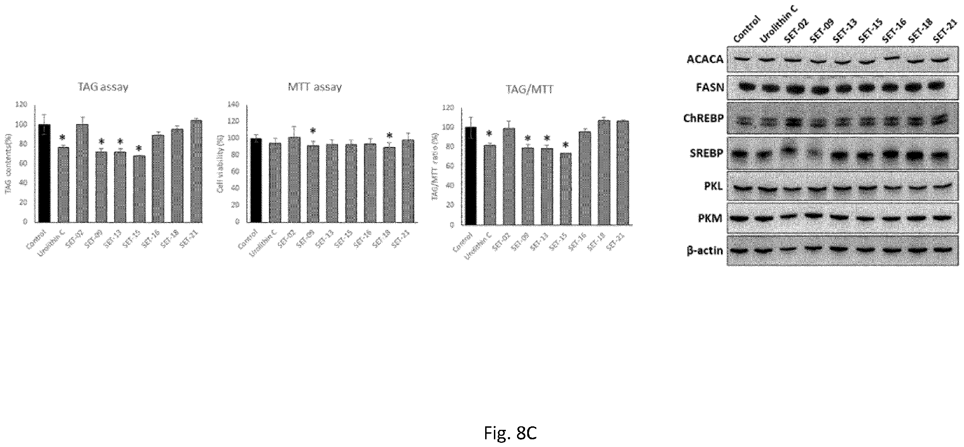

Fig 8 shows (A) PK activity in HepG2 WT and HepG2 PKM2 CRISPR KO protein lysate when treated with 10µM of SET-02, SET-09, SET-13, SET-15, SET-16, SET-18, SET-21 and Urolithin C. were observed pyruvate kinase activity on HepG2 WT and HepG2 PKM2 CRISPR KO cells protein lysate. Urolithin C was treated as a positive control. Generated pyruvate (O.D value at 570nm) is shown with a histogram. (B) PK activity in HepG2 WT and HepG2 PKM2 CRISPR KO cells when treated with 10 µMof SET-02, SET-09, SET-13, SET-15, SET-16, SET-18 and SET-21 for 4h. Generated pyruvate (O.D value at 570nm) is shown with a histogram. Data are represented as mean ± SD, *p < 0.05, Student's t test. (C) TAG assay, cell viability (MTT assay) and TAG/MTT of the HepG2 WT steatosis model was treated with 10µM of SET-02, SET-09, SET-13, SET-15, SET-16, SET-18, SET-21 and Urolithin C for one week. The expression of DNL involved steatosis proteins. Data are represented as mean ± SD, *p < 0.05, Student's t test.

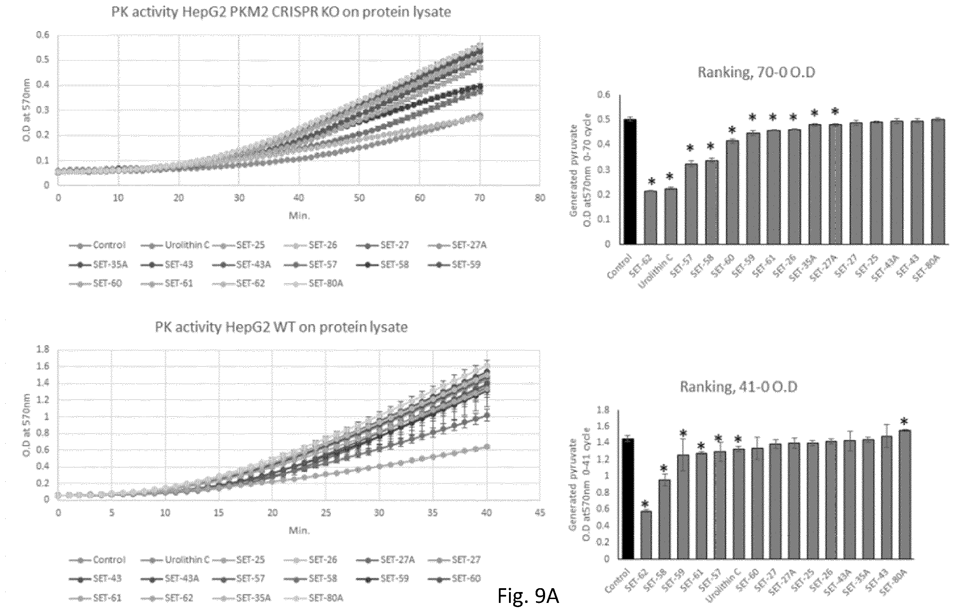

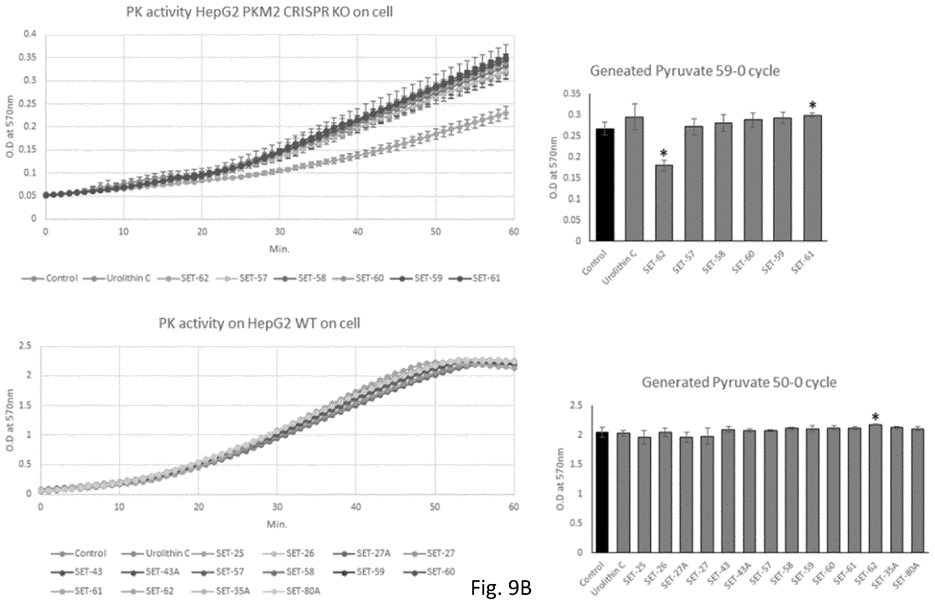

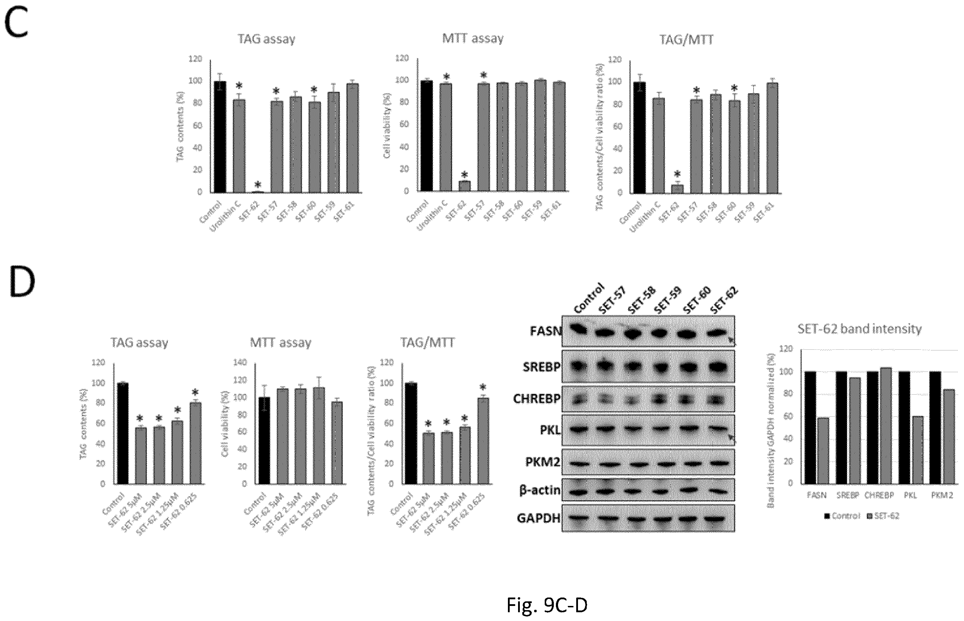

Fig 9 shows (A) PK activity in HepG2 WT and HepG2 PKM2 CRISPR KO protein lysate when treated with 10µM of SET-25, SET-26, SET-27, SET-27A, SET-35A, SET-43, SET-43A, SET-57, SET-58, SET-59, SET-60, SET-61, SET-62, SET-80A and UrolithinC. Generated pyruvate (O.D value at 570nm) is shown with a histogram. (B) PK activity in HepG2 WT when treated with 20µM of SET-25, SET-26, SET-27, SET-27A, SET-35A, SET-43, SET-43A, SET-57, SET-58, SET-59, SET-60, SET-61, SET-62, SET-80A and Urolithin C for 4 h. PK activity in HepG2 PKM2 CRISPR KO when treated with 20µM of SET-62, SET-57, SET-58, SET-60, SET-59, and SET-61 for 4 h. Generated pyruvate (O.D value at 570nm) is shown with a histogram. (C) HepG2 WT steatosis model was treated with 10µM of SET-62, SET-57, SET-58, SET-60, SET-59, and SET-61 for one week. After one week, TAG content and cell viability were measured. Western blot analysis for one-week steatosis HepG2 WT of DNL involved steatosis proteins. (D) DNL steatosis model was treated with 5µM, 2.5µM, 1.25µM or 0.625µM of SET-62 for one week. TAG and cell viability are shown. Data are represented as mean ± SD, *p < 0.05, Student's t test.
Fig 10 shows (A) PK activity in HepG2 WT and HepG2 CRISPR PKM KO cells treated with 20µM SET-62 for 4 h. Generated pyruvate during assay is visualized in a histogram. (B) Cetsa (Cellular thermal shift) for PKM2 and PKL. (C) Western blot analysis of DNL involved steatosis proteins after a DNL steatosis model was treated with 5µM SET-62is model. SET-62 band intensity is also shown for DNL involved steatosis proteins. Data are represented as mean ± SD, *p < 0.05, Student's t test.

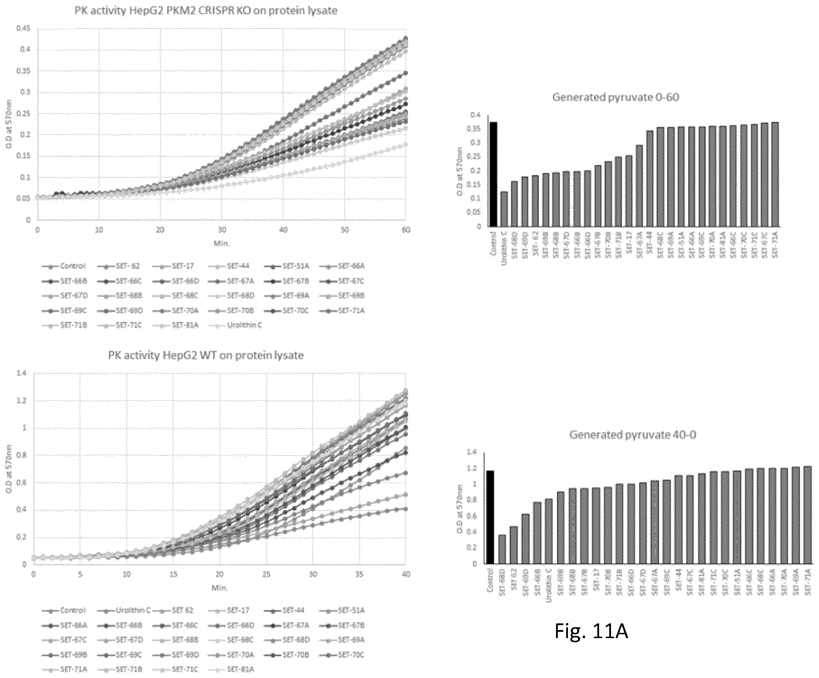

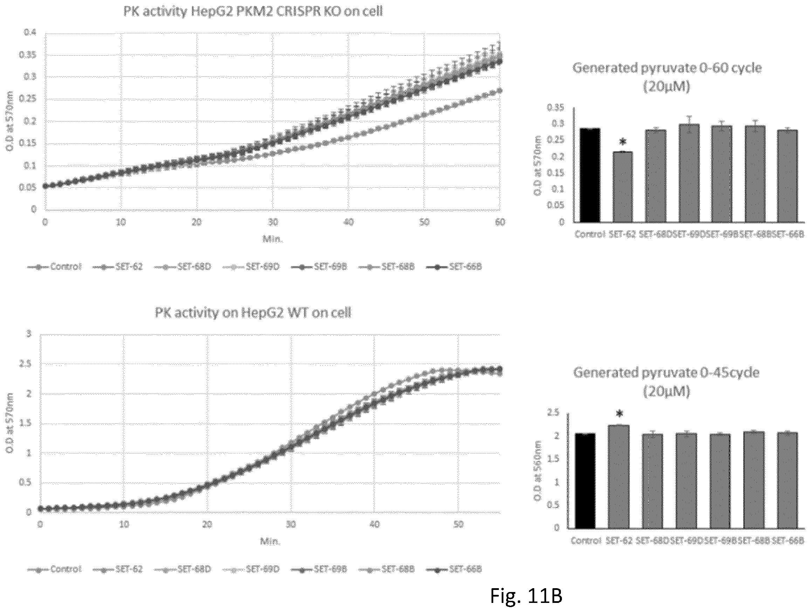

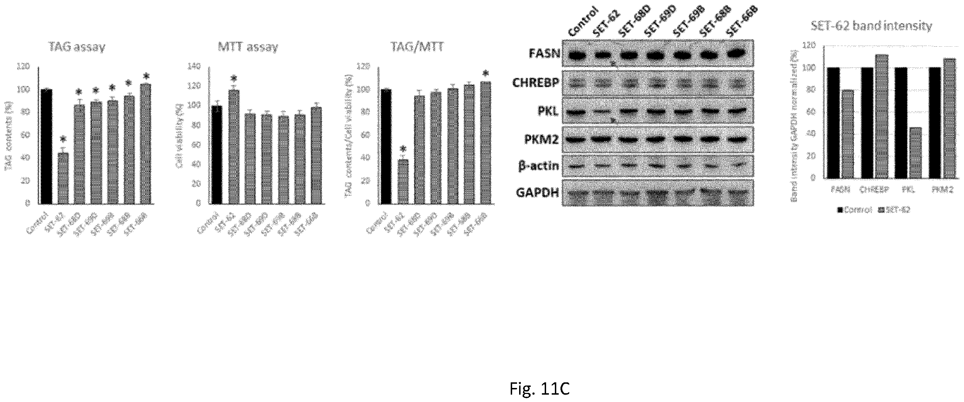

Fig 11 shows (A) PK activity in HepG2 WT and HepG2 PKM2 CRISPR KO protein lysate when treated with 10µM of SET-17, SET-44, SET-51A, SET-66A, SET-66B, SET-66C, SET-66D, SET-67A, SET-67B, SET-67C, SET-67D, SET-68B, SET-68C, SET-68D, SET-69A, SET-69B, SET-69C, SET-69D, SET-70A, SET-70B, SET-70C, SET-71A, SET-71B, SET-71C, SET-81A, SET-62 and Urolithin C. Generated pyruvate (O.D value at 570nm) is shown with a histogram. (B) PK activity in HepG2 WT and HepG2 PKM2 CRISPR KO cells were treated with SET-68D, SET-69D, SET-69B, SET-68B, SET-66B and SET-62. Generated pyruvate (O.D value at 570nm) is shown with a histogram. (C) DNL steatosis model was treated with 10µM of SET-68D, SET-69D, SET-69B, SET-68B, and SET-66B for one week at. After one week, TAG and Cell viability were measured. Western blot analysis for one-week steatosis HepG2 WT of DNL involved steatosis proteins is shown as well as SET-62 band intensity for DNL involved steatosis proteins. Data are represented as mean ± SD, *p < 0.05, Student's t test.

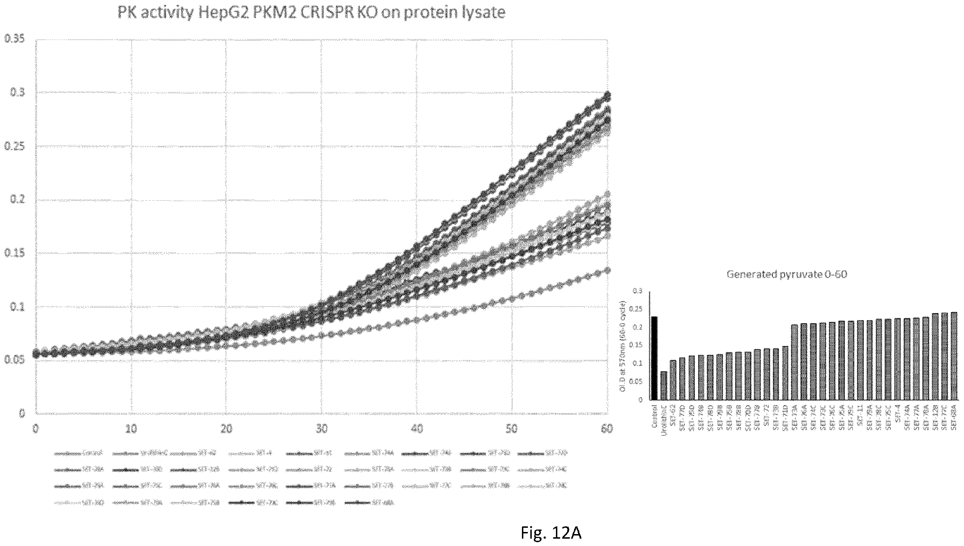

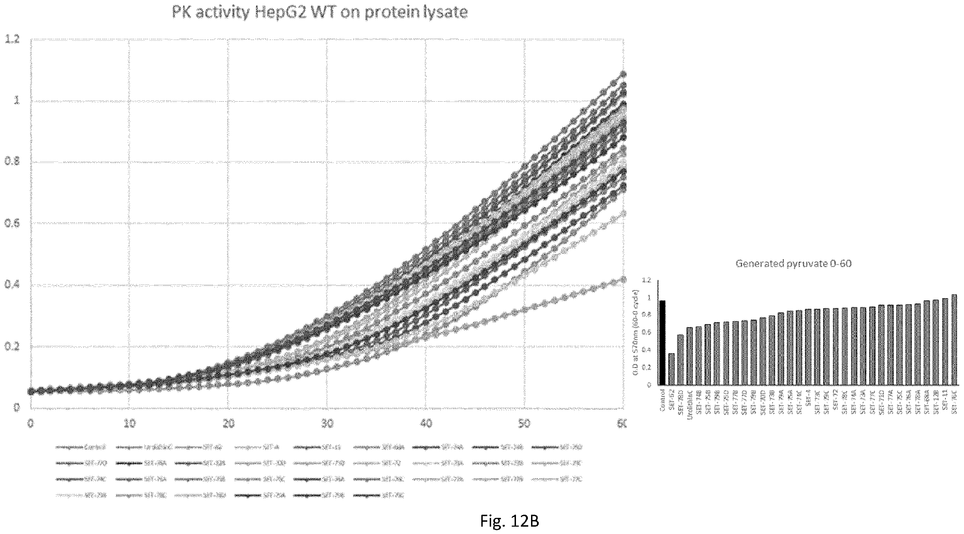

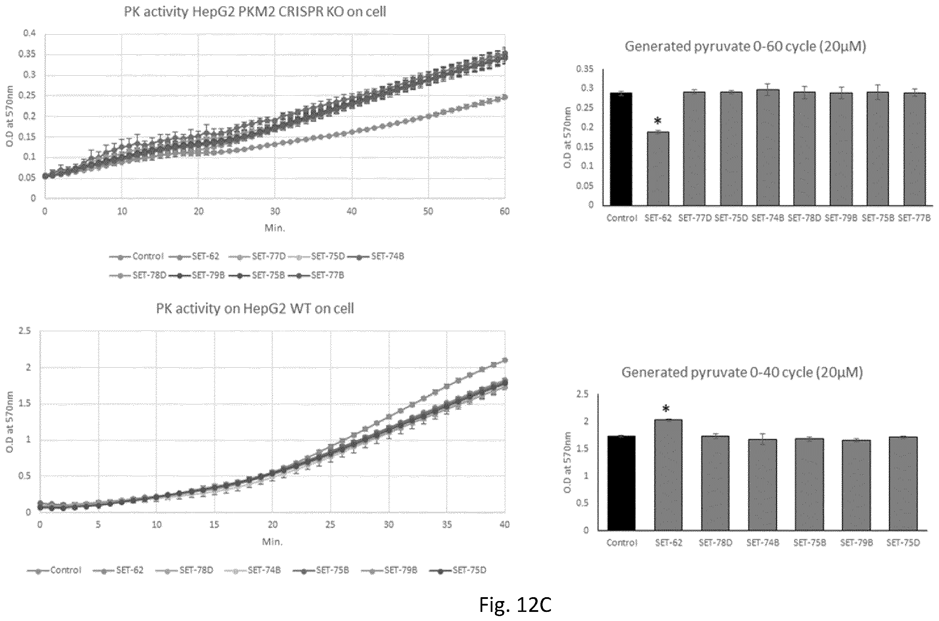

Fig 12 shows (A) PK activity in HepG2 PKM2 CRISPR KO cells protein lysate when treated with 10µM of urolithin-C, SET-62, SET-4, SET-11, SET-74A, SET-74B, SET-75D, SET-77D, SET-78A, SET-70D, SET-12B, SET-71D, SET-72, SET-73A, SET-73B, SET-73C, SET-74C, SET-75A, SET-75C, SET-76A, SET-76C, SET-77A, SET-77B, SET-77C, SET-78B, SET-78C, SET-78D, SET-79A, SET-75B, SET-79C, SET-79B and SET-68A (showed in this order in the graph). Generated pyruvate (O.D value at 570nm) is shown in a histogram. (B) PK activity in HepG2 WT cells protein lysate when treated with 10µM of Urolithin-C, SET-62, SET-4, SET-11, SET-68A, SET-74A, SET-74B, SET-75D, SET-77D, SET-78A, SET-12B, SET-70D, SET-71D, SET-72, SET-73A, SET-73B, SET-73C, SET-74C, SET-75A, SET-75B, SET-75C, SET-76A, SET-76C, SET-77A, SET-77B, SET-77C, SET-78B, SET-78C, SET-78D, SET-79A, SET-79B, SET-79C (shown in this order). Generated pyruvate (O.D value at 570nm) is shown in a histogram.
(C) PK activity in HepG2 PKM2 CRISPR KO and HepG2 WT cells when treated with SET-77D, SET-75D, SET-74B, SET-78D, SET-79B, SET-75B, SET-77 and SET-62. HepG2 WT cells were treated with SET-78D, SET-74B, SET-75B, SET-79B, SET-75D and SET-62. Generated pyruvate (O.D value at 570nm) is shown in a histogram. Data are represented as mean ± SD, *p < 0.05, Student's t test.

### DETAILED DESCRIPTION

PKL - liver pyruvate kinase has been identified as a target to halt the progression of the NAFLD [2,6,7]. The present inventors, therefore, started an extensive program aimed to identify new compounds to inhibit PKL [12-14]. These efforts resulted in the identification of a class of PKL inhibitors based on ellagic acid, that show a non-competitive inhibition of the enzyme based on the interaction with an allosteric site [12,14]. Deconstruction and simplification of ellagic acid identified one of its metabolites, urolithin C, as a more soluble and bioavailable PKL inhibitor. The present inventors thus realized that there is a possibility to develop and optimize small compounds, which may be used in the treatment of fatty liver disease and hepatocellular carcinoma (HCC). The development/optimization was based on alteration of the chemical structure of urolithin C in order to improve the efficacy of small compounds in regard to PKL inhibition and reduction of triglyceride (TAG) levels.

According to a first aspect of the present disclosure there is provided a compound according to formula (I) 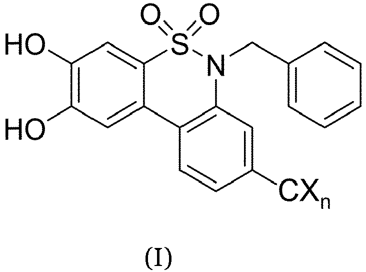 or a pharmaceutically acceptable salt or prodrug thereof wherein X is a halogen and n=1-3.

Treatment of HepG2 cells with a compound according to formula (I) results in inhibition of the expression of PKL and a reduction in TAG levels. A compound according to formula (I) has also been shown to pass the cell wall and enter the cells, which is crucial in order for the compound to be used in the treatment of NAFLD. Thus, a compound according to formula (I) is particularly well suited for use in pharmacological treatment of NAFLD.

X is a halogen and may be selected from chloride, bromide, fluoride and iodine, preferably X is fluoride.

In one embodiment, n is 2 or 3. Thus, the -CXₙ group may be -CF₃, -CBr₃, -CCl₃ or -CI₃.

In another embodiment, the -CXₙ group is -CF₂ or -CF₃.

According to a particularly preferred embodiment, the compound is a compound according to formula (II)

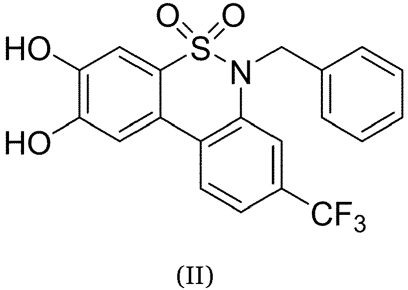

Compound SET-62 in the examples section is a compound according to formula (II). Treatment with this compound is associated with a particularly high reduction in TAG levels and expression levels of PKL and fatty acid synthase (FASN). The compound according to formula (II) is therefore particularly well suited for use in the treatment of NAFLD.

It is further believed that a compound according to formula (II) has a suitable metabolic stability and good cell-permeability.

A compound according to the first aspect has been found to have a selective inhibition of the PKL isomer compared to other isomers. As an example, the compound SET-62 as disclosed in the Examples section has been shown to have an inhibitory effect on PKL activity while having an activating effect on PKM2 activity.

Furthermore, it has been shown that a compound according to the first aspect may have a concentration dependent toxicity in HepG2 cells, wherein at low doses the compound may reduce the TAG levels while not influencing the cell viability. However, at higher doses both a reduction in TAG levels and cell viability may be obtained. Thus, at higher doses a compound according to the first aspect may be used as a medicament for treating hepatocellular carcinoma (HCC).

According to a second aspect, a pharmaceutical composition comprising a compound according to the first aspect is provided.

According to a third aspect, a compound according to the first aspect or a pharmaceutical composition according to the second aspect used in a method of treatment of fatty liver disease or hepatocellular carcinoma (HCC) is provided.

The method of treatment with the compound or pharmaceutical composition may comprise oral administration of the compound.

The fatty liver disease is preferably non-alcoholic fatty liver disease (NAFLD), which may have progressed to non-alcoholic steatohepatitis (NASH).

### EXAMPLES

### Methods

### Molecular Docking

Computational docking studies were performed using the MOE v. 2019.01 [15]. The protein structure with the PDB ID code: 7FS5 was prepared using the protein preparation module in MOE by the addition of missing atoms and residues, correction of bond order and formal charge and adjustment of tautomer [16]. System was protonated using the protonate 3D algorithm and generalized Born volume integral (GB/VI) was used as the electrostatics function, with a value of 80 as dielectric constant. The electrostatics and van der Waals cut off were set as 10 and 15 Å, respectively. Protein was charged and minimized with the application of AMBER10:EHT force field implemented in a MOE software.

All of the compounds were built using ChemBioDraw Ultra 14.0, charged and minimized by MMFF94x force field, along with the adjustment of hydrogens and lone pairs [17]. The dataset was subjected to energy minimization using default RMS gradient of 0.1 kcal/mol/Å². Following protonation and minimization, the compounds were saved in the MOE database format.

The allosteric site, which lies between two chain B and D, was considered for docking to include all atoms within 5 Å of cognate ligand. Prior to executing the docking, benchmarking of different combination of scoring and placement methods in MOE Dock was performed to find out the most suitable combination of algorithm and scoring functions for the target protein. The most reliable results were obtained by induced fit docking procedure along with Triangle Matcher algorithm as placement method and LondonDG as initial scoring and GBVI/WSA dG as re-scoring method. For each compound, 10 individual docking runs were conducted, and 100 conformations were generated. The best-ranked solution of each compound was further assessed for binding mode analysis.

Molecular docking was performed to select the target compounds which were then synthesised, and their effectiveness analysed in vitro (see below).

### Biological Assays Procedure

### Cell culture experiments and in vitro steatosis induction

HepG2 wild type cells were purchased from genome engineering company Synthego. Cells were maintained with growth media RPMI 1640 (R2405, Sigma-Aldrich) and supplemented with 10 % fetal bovine serum (FBS, F7524, Sigma-Aldrich), 1 % penicillin-streptomycin (P/S media, P4333, Sigma-Aldrich). Steatosis induction media was prepared as DMEM high glucose (D0819, Sigma-Aldrich) with 10% FBS, 1% P/S media supplemented with 10µg/ml insulin (I9278, Sigma-Aldrich), and 10µM T0901317 (T2320, Sigma-Aldrich) for one week. HepG2 cells were seeded 6×104 cells per well into a 96-well plate for TAG and MTT assay after steatosis and 1×106 cells per well into 6-well plate for western blot analysis after steatosis. HepG2 cells were incubated with steatosis induce media (SM) for one week by 3 day + 2 day + 2 day with total three times media exchange.

### TAG Measurement and MTT assay

HepG2 cells were seeded at 6×104 cells per well into 96-well plate and induced steatosis for one week with designated concentration of small molecules (this will be referred to as a DNL steatosis model). After steatosis induction, cells were washed with 200 µl of PBS and triglyceride contents was measured by Triglyceride Assay Kit - Quantification (ab65336, Abeam) following manufacturer's instruction. Optical density (O.D) values were detected with microplate reader at 570 nm (Hidex Sense Beta Plus).

5 mg/ml MTT (M2128, Sigma-Aldrich) solution in PBS (10 µL) was added to each well for MTT assay. After 1 hr, MTT solution and all media were removed from the wells. 100 µl of DMSO was added and mixed to dissolve the formazan crystals. Cell viability was analysed by measuring the absorbance of the dissolved formazan in a microplate reader at a wavelength of 570 nm with microplate reader (Hidex Sense Beta Plus). DMSO was used as control.

### Pyruvate kinase activity

HepG2 WT and HepG2 KO cells were lysed, and protein lysate was prepared with CelLytic M (C2978, Sigma-Aldrich) lysis buffer. Protein lysate was treated with 10 µM of the drugs and the pyruvate kinase activity on protein lysate was measured using Pyruvate Kinase Assay Kit (ab83432, abeam) following manufacturer's instruction. 2×104 HepG2 WT cells and 1×105 HepG2 KO cells were seeded into a 96-well plate for measuring pyruvate kinase activity in the cells. The cells were treated with 10-20µM of drugs for 4h and washed with 250µl PBS. Cells were lysed with 50 µl PK assay buffer (ab83432, abcam) and start reaction and kinetic O.D measurement was performed on 50µl PK assay buffer containing the assay component (ab83432, abcam). O.D values were measured with microplate reader (Hidex Sense Beta Plus) at 570nm wavelength. DMSO was used as control.

### Western blot

After steatosis induction with designated concentration of compounds, whole cell lysate was prepared with CelLytic M (C2978, Sigma-Aldrich) buffer. SDS PAGE was performed on Mini-PROTEAN^{®} TGX^{™} Precast Gels (Bio-Rad) and transferred using Trans-Blot^{®} Turbo^{™} Transfer System (Bio-Rad). FASN (ab22759, abcam), ACACA (NBP2-55439, Novus), ChREBP (92809, abcam), SREBP-1C (PA1 337, Invitrogen), PKL (06653, Sigma), PKM (4053S, Cell signalling), β-actin(ab8227, abcam), GAPDH (sc-47724, SANTA CRUZ) were blotted overnight as a primary antibodies. Secondary antibodies, Goat Anti-Rabbit HRP (ab205718) and goat antimouse IgG-HRP(sc2005, Santa Cruz Biotechnology, Inc.) were blotted for one hour. Protein band was detected with ImageQuantTMLAS 500 (29-0050-63, GE). DMSO was used as control.

### Cellular thermal shift assay (Cetsa)

HepG2 WT cells were trypsinized and transferred into 1.5ml tube at a concentration of 500,000 cells per ml. The cells were treated with 10µM and 20µM SET-62 in an 1.5 ml tube and incubated for 2 h at 37°C in an CO₂ incubator. The tubes were exposed to heat shock at 60°C for 5 min with a pre-heated heat block. After centrifugation of the heated cells at 13,000 rpm for 1 min, the cells were lysed with 50 µl of Native lysis Buffer (ab156035, abeam). Centrifugation was performed again to purify soluble proteins in the lysed solution and 9 µl of each group were analysed using western blot. DMSO was used as control.

### Synthesis of target compounds

### General Experimental

All reactions were performed with oven-dried glassware and under an inert atmosphere (nitrogen) unless otherwise stated. All reagents were obtained from commercial supplier Sigma-Aldrich and used without further purification. Dried solvents were obtained from commercial supplier Sigma Aldrich. Organic solutions were concentrated under reduced pressure on a Heidolph rotary evaporator. Reactions were monitored by LC-MS (Thermo Fisher TSQ Series, Athena C18-WP,100 Å, 2.1×50 mm, 3 µm); Water:MeOH (0.01 formic acid)) or by Thin-Layer Chromatography (TLC) and TLC analysis was done using silica gel pre-coated aluminum plates (Kieselgel 60, 254, E. Merck, Germany). The chromatograms were visualized using ultraviolet light (254 and 366 nm) and stained with vanillin dips or aqueous potassium permanganate solution. ¹H NMR spectra were recorded on Avance Bruker 500 MHz spectrometer in CDCl₃ and DMSO-*d₆*. ¹³C NMR spectra were recorded in deuterated solvents on Bruker spectrometer at 126 MHz, with the central peak of the deuterated solvent as the internal standard. The ¹H NMR spectra are reported as δ/ppm downfield from tetramethyl silane (multiplicity, number of protons, coupling constant J/Hz). The ¹³C NMR spectra are reported as δ/ppm. All chemical shifts are reported in parts per million (ppm) relative to the residual solvent peak. The following abbreviations are used to denote signal patterns: (s) singlet, (d) doublet, (t) triplet, (q) quartet, (m) multiplet, and (br) broad unless otherwise noted. Flash-column chromatography was performed on PuriFlash XS 520 Plus Flash chromatography system (Interchim) with built-in UV-detector, ELSD-detector and fraction collector with Interchim silica gel columns.

*General procedure A:* sulfonamides preparation. 2-bromo-4,5-dimethoxybenzene-1-sulfonyl chloride 2 (1.0 eq.) was added to the stirred solution of corresponding amine (1.2 eq.) and DIPEA in dry DCM (20 mL) cooled to 0°C. Reaction was allowed to warm to r.t. and stirred until complete consumption of the starting material was determined by TLC (Hexane:EtOAc/3:2), 1-2h. Reaction is quenched with water (20 mL) and crude product extracted with DCM (3×20 mL). Combined organic phases were washed with 1M HCl (3×60 mL) and brine (3×60 mL), and then dried over anhydrous Na₂SO₄. Solvent was removed under reduced pressure to afford crude sulfonamides, which was purified by trituration with methanol/purified by flash chromatography using Hexane/EtOAc (gradient) to afford sulfonamides.

*General procedure B:* Cross coupling reaction. Biaryl sulfonamides were prepared according to the slightly modified described procedure. [18] Sulfonamides (1.0 eq.), appropriate boronic acid (1.5 eq.), potassium carbonate (4.0 eq.) and Pd(PPh₃)₄ (0.03 eq.) were suspended in the mixture of toluene:ethanol:water/5:2:1 (13 mL). Sequential applying of N₂ flow and vacuum were used to degas the mixture before heating in a microwave reactor at 120 °C for 1h. The crude product was extracted with ethyl acetate (3 × 30 mL). The organic phase was dried over sodium sulfate. The mixture was then filtered through the bed of celite and solvent was removed under reduced pressure. The residue was purified by flash chromatography using Hexane/EtOAc (gradient) to afford the biaryl sulfonamides.

*General procedure C:* cyclization of secondary sulfonamide. Cyclization was achieved according to the procedure described in the literature [19]. Appropriate sulfonamide (1 eq.), PIDA (1.10 eq.), I₂ (1.10 eq.) and K₂CO₃ (1.50 eq.) were suspended in DCM (20 mL). The dark red solution was stirred at 35 °C for 3h, until almost complete conversion was determined by TLC (3 % EtOAc in DCM). Reaction is quenched with sat. aq. Na₂S₂O₅, and mixture was stirred at r.t. until discoloration of the solution. Crude product was extracted with DCM and purified via flash chromatography (DCM/EtOAc, gradient, 0->1% EtOAc) and triturated with MeOH to afford sultams.

*General procedure D:* Deprotection of methoxy groups. To a solution of appropriate protected compound (1 eq.) cooled to 0°C in DCM (5-10 mL) was added BBr₃ (1M in DCM, 5 eq. per methoxy group). Reaction was allowed to warm up to room temperature and stirred (3-12h). LC/MS analysis revealed complete consumption of the starting material and formation of N-benzyl or N-fluoro benzyl sultam as a main product alongside with a small to medium amount of corresponding debenzylated sultam. Reaction is cooled to 0°C and quenched with water and mixture extracted with diethyl ether (3 × 50 mL). Combined org. phases were dried over Na₂SO₄, evaporated and Crude was purified via reverse phase column to afford compound.

*General procedure E:* Deprotection of benzyl groups. To achieve complete cleavage of the benzyl group, methanesulfonic acid (1 mL) was added to the mixture and reaction stirred for one hour at the r.t.. Reaction is quenched with water (50 mL) and crude product extracted with EtOAc (3 × 50 mL). Combined organic phases were dried over Na₂SO₄ and evaporated. Crude was purified via reverse phase column to afford compound.

*General procedure F:* Azide preparation. A solution of alkyl bromide (1 equiv.) and sodium azide (2 equiv.) in EtOH/H₂O (2:1) was stirred at room temperature for 2h. The mixture was evaporated under reduced pressure then extracted with diethyl ether and water. The combined organic extracts were dried over magnesium sulphate and evaporated to dryness under reduced pressure to give the desired product.

*General procedure G:* CuAAC reactions. To a solution of Alkyne (1 eq.) and copper sulfate (0.75 eq.) in DCM/H₂O (2:1), sodium ascorbate (0.25 eq.) was added. The mixture was stirred at room temperature for 20 min. Then appropriate azide (1.5 eq. ) was added and reaction was stirred for further 6-16 h. The mixture was then extracted with Ethylacetate and water. The combined organic extracts were dried over magnesium sulphate and evaporated to dryness under reduced pressure to yield the desired products. Compounds were purified by flash chromatography.

### General Procedure for the Synthesis of 2-bromo-4,5-dimethoxybenzenesulfonyl chloride (2)

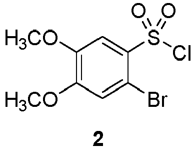

4-Bromoveratrole 1 (4.0 ml, 27.8 mmol) was added in dropwise over 20 min to a well stirred flask containing chlorosulfonic acid (8 ml) cooled to 0 °C. The black mixture was then stirred for an additional 30 min before cautious addition to ice (150 ml). After melting DCM (100ml) was added and the phase were separated. The aqueous phase was extracted with DCM (100 ml) and the combined organic phases were dried over MgSO₄. Filtration and solvent removal under reduced pressure gave the sulfonyl chloride 2 (7.17 g 82%) as a white solid which was further purified by recrystallisation from diethylether. m.p. 78 °C; ¹H NMR (400 MHz, CDCl₃) δ 3.94 (s, 3H), 3.99 (s, 3H), 7.18 (s, 1H), 7.59 (s, 1H); ¹³C NMR (100 MHz, CDCl₃) δ 56.5, 56.7, 112.5, 113.0, 117.7, 134.6, 147.8, 154.1.

### Synthesis of N-benzyl-2-bromo-4,5-dimethoxybenzenesulfonamide (SET-04)

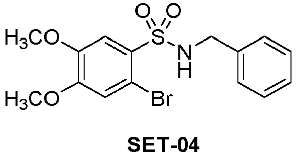

2 (10.0 g, 31.69 mmol) was added to a solution of benzylamine (4.15 mL, 38.03 mmol) and DIPEA (16.45 mL, 95.07 mmol) in DCM (30 mL) cooled to 0 °C. Reaction was allowed to warm to r.t. and was stirred for 6 hours, until complete conversion of the starting material has been confirmed by TLC (3% EtOAc in DCM). Reaction was quenched with water (30 mL). Crude product was extracted with DCM (3 × 30 mL). Combined organic phases were washed with 1M HCl (3 × 50 mL), dried over Na₂SO₄ and evaporated to obtain the white solid SET-04 (10.7 g, 90%). ¹H NMR (400 MHz, DMSO-d6) δ 8.24 (s, 1H, NH), 7.38 (s, 1H, Ar), 7.26 (s, 1H, Ar), 7.25-7.15 (m, 5H, Ar), 4.08 (s, 2H, CH₂), 3.84 (s, 3H, CH₃), 3.76 (s, 3H, CH₃); ¹³C NMR (101 MHz, DMSO-*d₆*) δ 151.7, 147.3, 137.6, 131.5, 128.0, 127.6, 127.0, 117.4, 113.3, 110.6, 56.3, 55.8, 46.1.

### General procedure for the synthesis of biaryl sulfonamides (Cross coupling)

Biaryl sulfonamides were prepared according to the slightly modified described procedure.¹ Compound SET-04 (1.0 eq.), appropriate boronic acid (1.5 eq.), potassium carbonate (4.0 eq.) and Pd(PPh₃)₄ (0.03 eq.) were suspended in the mixture of toluene:ethanol:water/5:2:1 (13 mL). Sequential applying of N₂ flow and vacuum were used to degas the mixture before heating in a MW reactor at 120 °C for 1h. The crude product was extracted with ethyl acetate (3 × 30 mL). To the organic phase was dried over sodium sulfate. The mixture was then filtered through the bed of celite and solvent removed under reduced pressure. The residue was purified by flash chromatography using Hexane/EtOAc (gradient) to afford the biaryl sulfonamides.

### Synthesis of N-benzyl-3',4,5-trimethoxy-[1,1'-biphenyl]-2-sulfonamide (SET-69A)

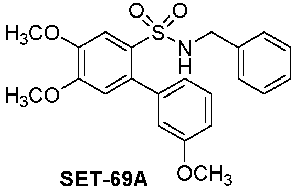

SET-04 (400 mg, 1.03 mmol), 3-Methoxybenzeneboronic acid (334 mg, 1.54 mmol), K₂CO₃ (560 g, 4.12 mmol) and Pd(PPh₃)₄ (34 mg, 0.03 mmol) were used according to the general procedure B to afford compound SET-69A as a white solid (80%). ¹H NMR (500 MHz, DMSO-*d₆*) δ 7.65 (t, *J* = 6.3 Hz, 1H), 7.44 (s, 1H), 7.27 (m, 3H), 7.20 (m, 3H), 6.94 (m, 3H), 6.82 (s, 1H), 5.75 (s, 1H), 3.87 (d, *J* = 6.2 Hz, 2H), 3.82 (d, *J* = 7.5 Hz, 6H), 3.75 (s, 3H); ¹³C NMR (126 MHz, DMSO-*d₆*) δ 158.80, 151.05, 147.79, 141.46, 138.40, 134.50, 131.13, 129.00, 128.64, 128.07, 127.56, 122.37, 115.79, 115.55, 113.27, 111.76, 56.36, 56-33, 55-43, 46.51.

### Synthesis of N-benzyl-4,4',5-trimethoxy-[1,1'-biphenyl]-2-sulfonamide (SET-67A)

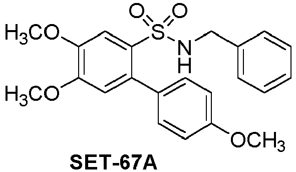

SET-04 (300 mg, 0.58 mmol), 4-Methoxybenzeneboronic acid (170 mg, 1.16 mmol), K₂CO₃ (400 mg, 2.9 mmol) and Pd(PPh₃)₄ (34 mg, 0.02 mmol) were used according to the general procedure B to afford compound SET-67A as white solid (74%). ¹H NMR (500 MHz, DMSO-*d₆*) *δ* 7.57 (t, *J* = 6.1 Hz, 1H), 7.43 (s, 1H), 7.30 (m, 2H), 7.26 (dd, *J* = 1.2, 7.1 Hz, 2H), 7.23 (m, 1H), 7.18 (m, 2H), 6.93 (m, 2H), 6.78 (s, 1H), 3.85 (d, *J* = 5.8 Hz, 2H), 3.82 (s, 3H), 3.80 (s, 3H), 3.79 (s, 3H) ; ¹³C NMR (126 MHz, DMSO-d6) δ 159.04, 151.06, 147.54, 138.44, 134.54, 132.36, 131.22, 131.13, 128.62, 128.07, 127.54, 116.15, 115.86, 115.05, 113.41, 111.83, 56.31, 55.56, 46.47.

### Synthesis of N-benzyl-4,5-dimethoxy-4'-(trifluoromethyl)-[1,1'-biphenyl]-2-sulfonamide (SET-26)

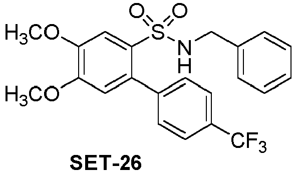

SET-04 (500 mg, 1.29 mmol), 4-Trifluoromethylbenzeneboronic acid (293 mg, 1.93 mmol), K₂CO₃ (713 mg, 5.16 mmol) and Pd(PPh₃)₄ (57 mg, 0.5 mmol) were used according to the general procedure B to afford compound SET-26 as white solid (72%). ¹H NMR (500 MHz, DMSO- *d₆*) δ 7.93 (t, *J* = 6.3 Hz, 1H), 7.73 (d, *J* = 8.1 Hz, 2H), 7.57 (d, *J* = 8.0 Hz, 2H), 7.45 (s, 1H), 7.23 (m, 5H), 6.86 (s, 1H), 3.92 (d, *J* = 6.2 Hz, 2H), 3.82 (d, *J* = 1.9 Hz, 6H); ¹³C NMR (126 MHz, DMSO-*d₆*) δ 151.17, 148.24, 144.50, 138.27, 133.10, 131.20, 130.79, 128.63, 128.10, 128.07, 127.59, 124.79, 124.76, 124.73, 115.37, 111.75, 56.45, 56.34, 46.46.

### Synthesis of N-benzyl-3',4,4',5-tetramethoxy-[1,1'-biphenyl]-2-sulfonamide (SET-06)

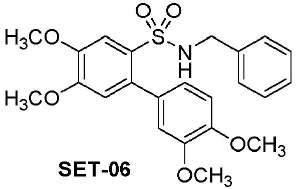

SET-04 (500 mg, 1.29 mmol), 3,4-Dimethoxybenzeneboronic acid (350 mg, 1.93 mmol), K₂CO₃ (710 mg, 5.16 mmol) and Pd(PPh₃)₄ (43 mg, 0.03 mmol) were used according to the general procedure B to afford compound SET-06 as white solid (92%). ¹H NMR (400 MHz, DMSO-*d₆*) *δ* 7.50 (t, J = 6.3 Hz, 1H, NH), 7.46 (s, 1H, Ar), 7.30 - 7.15 (m, 5H, Ar), 7.01 (d, J = 2.0 Hz, 1H, Ar), 6.96 (d, J = 8.3 Hz, 1H, Ar), 6.92 (dd, J = 8.2, 2.0 Hz, 1H, Ar), 6.84 (s, 1H, Ar), 3.86 (d, J = 6.2 Hz, 2H, CH₂), 3.83 (s, 3H, CH₃), 3.81 (s, 3H, CH₃), 3.78 (s, 3H, CH₃), 3.73 (s, 3H, CH₃); ¹³C NMR (101 MHz, DMSO-*d₆*) *δ* 150.6, 148.2, 147.6, 147.1, 137.9, 134.2, 132.0, 130.6, 128.2, 127.6, 127.1, 121.8, 115.4, 113.7, 111.5, 111.0, 55.9, 55.9, 55.5, 55.4, 46.0.

### Synthesis of N-benzyl-3',4,5,5'-tetramethoxy-[1,1'-biphenyl]-2-sulfonamide (SET-71A)

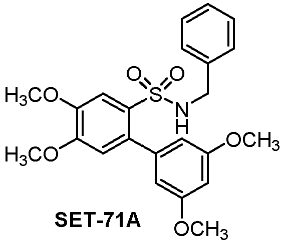

SET-04 (500 g, 1.29 mmol), 3,5-Dimethoxybenzeneboronic acid (460 mg, 2.5 mmol), K₂CO₃ (890 mg, 6.45 mmol) and Pd(PPh₃)₄ (50 mg, 0.05 mmol) were used according to the general procedure B to afford compound SET-71A as white solid (82%). ¹H NMR (500 MHz, DMSO-*d₆*) δ 7.63 (t, J = 6.3 Hz, 1H), 7.44 (s, 1H), 7.27 (m, 2H), 7.20 (m, 3H), 6.84 (s, 1H), 6.55 (d, J = 2.3 Hz, 2H), 6.49 (t, J = 2.3 Hz, 1H), 3.89 (d, J = 6.3 Hz, 2H), 3.83 (s, 3H), 3.81 (s, 3H), 3.74 (s, 6H); ¹³C NMR (126 MHz, DMSO-d6) δ 161.60, 159.96, 159.60, 151.05, 147.80, 141.93, 138.37, 134.48, 131.01, 128.65, 128.08, 127.58, 115.42, 111.78, 108.41, 99.69, 94.39, 91.93, 56.38, 56.34, 55-59, 55.38, 46.55.

### Synthesis N-benzyl-4,5-dimethoxy-3',5'-bis(trifluoromethyl)-[1,1'-biphenyl]-2-sulfonamide(SET-74A)

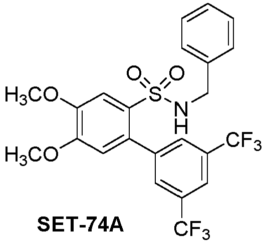

SET-04 (600 g, 1.55 mmol), 3,5-Ditrifluoromethylbenzeneboronic acid (700 mg, 3.10 mmol), K₂CO₃ (850 mg, 6.2 mmol) and Pd(PPh₃)₄ (80 mg, 0.07 mmol) were used according to the general procedure B to afford compound SET-74A as white solid (85%). ¹H NMR (500 MHz, Chloroform-*d*) δ 7.86 (s, 1H), 7.73 (d, J = 1.6 Hz, 2H), 7.64 (s, 1H), 7.24 (dd, J = 2.1, 5.1 Hz, 3H), 7.01 - 6.94 (m, 2H), 6.66 (s, 1H), 3.99 (s, 3H), 3.95 (d, J = 5.9 Hz, 2H), 3.93 (s, 3H).¹³C NMR (126 MHz, CDCl₃) δ 151.79, 148.57, 140.88, 135.63, 131.27, 131.23, 130.96, 130.44, 130.09, 130.06, 128.82 (2CH), 128.21, 127.99 (2CH), 124.19, 122.01, 121.97, 114.45, 112.70, 56.52, 56.48, 47.25.

### Synthesis N-benzyl-4,5-dimethoxy-4'-(trifluoromethoxy)-[1,1'-biphenyl]-2-sulfonamide (SET-75A)

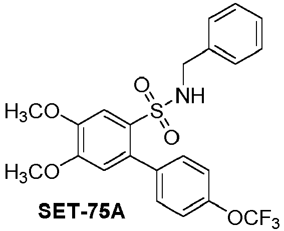

SET-04 (600 g, 1.55 mmol), 4-Trifluoromethoxybenzeneboronic acid (640 mg, 3.1 mmol), K₂CO₃ (820 mg, 6.0 mmol) and Pd(PPh₃)₄ (80 mg, 0.07 mmol) were used according to the general procedure B to afford compound SET-75A as white crystal solid (79%). ¹H NMR (500 MHz, Chloroform-*d*) δ 7.64 (s, 1H), 7.38 - 7.34 (m, 2H), 7.25 - 7.21 (m, 3H), 7.19 - 7.15 (m, 2H), 7.00 (dd, J = 3.2, 6.4 Hz, 2H), 6.70 (s, 1H), 3.98 (s, 3H), 3.91 (s, 3H), 3.84 (d, J = 6.2 Hz, 2H), 3.71 (t, J = 6.2 Hz, 1H). ¹³C NMR (126 MHz, CDCl₃) δ 151.57, 149.16, 148.11, 137.26, 135.83, 132.51, 131.15 (2CH), 130.03, 128.68 (2CH), 128.06, 127.87 (2CH), 120.43, 119.37, 119.26, 114.57, 112.37, 56.44, 56.34, 47.20.

### Synthesis N-benzyl-3'-fluoro-4,4',5-trimethoxy-[1,1'-biphenyl]-2-sulfonamide (SET-77A)

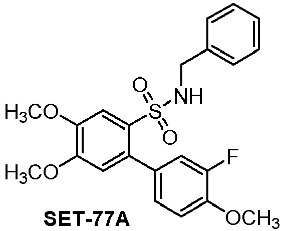

SET-04 (600 g, 1.55 mmol), (3-fluoro-4-methoxyphenyl) boronic acid (520 mg, 3.1 mmol), K₂CO₃ (850 mg, 6.2 mmol) and Pd(PPh₃)₄ (80 mg, 0.07 mmol) were used according to the general procedure B to afford compound SET-77A as white solid (78%). ¹H NMR (500 MHz, Chloroform-d) δ 7.59 (s, 1H), 7.22 - 7.16 (m, 3H), 7.13 - 7.08 (m, 1H), 6.99 (dd, J = 2.9, 6.6 Hz, 3H), 6.96 (dd, J = 2.2, 11.8 Hz, 1H), 6.87 (t, J = 8.6 Hz, 1H), 6.66 (s, 1H), 3.93 (s, 3H), 3.87 (s, 3H), 3.82 (s, 3H), 3.80 (d, J = 6.0 Hz, 2H), 3.76 (s, 1H). ¹³C NMR (126 MHz, CDCl₃) δ 152.55, 151.61, 150.58, 148.06, 147.88, 136.00, 132.54, 132.25, 131.15, 130.11, 128.76, 128.68, 128.13, 125.92, 117-53, 114-76, 112.88, 112.39, 56.52, 56.41, 56.34, 47-42.

### Synthesis N-benzyl-4,4',5-trimethoxy-3'-(trifluoromethyl)-[1,1'-biphenyl]-2-sulfonamide (SET-78A)

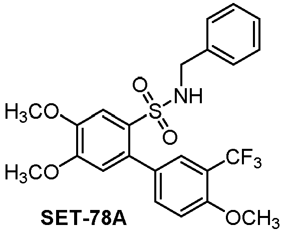

SET-04 (600 g, 1.24 mmol), (4-methoxy-3-(trifluoromethyl) phenyl) boronic acid (540 mg, 2.48 mmol), K₂CO₃ (685 mg, 4.96 mmol) and Pd(PPh₃)₄ (71 mg, 0.06 mmol) were used according to the general procedure B to afford compound SET-78A as white solid (82%). ¹H NMR (500 MHz, Chloroform-d) δ 7.68 - 7.54 (m, 2H), 7.40 (d, J = 2.2 Hz, 1H), 7.21 (dd, J = 2.5, 3.9 Hz, 3H), 7.08 - 6.93 (m, 3H), 6.67 (s, 1H), 3.96 (s, 3H), 3.90 (s, 3H), 3.88 (s, 3H), 3.82 (d, J = 6.1 Hz, 2H), 3.71 (t, J = 6.2 Hz, 1H).¹³C NMR (126 MHz, CDCl3) δ 157.53, 151.74, 148.14, 135.73, 132.54, 130.25, 130.19, 128.82, 128.19, 128.17, 127.83, 127.75, 124.47, 122.30, 118.55, 114-90, 112.59, 111.65, 77.16, 56.55, 56.47, 56.17, 47.48.

### Synthesis N-benzyl-2-(6-chloro-2-methoxypyridin-3-yl)-4,5-dimethoxybenzenesulfonamide(SET-79A)

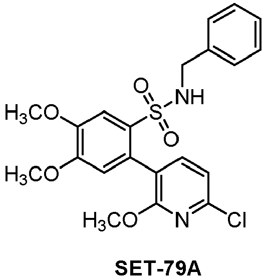

SET-04 (400 g, 1.03 mmol), (6-chloro-2-methoxypyridin-3-yl) boronic acid (380 mg, 2.06 mmol), K₂CO₃ (560 mg, 4.12 mmol) and Pd(PPh₃)₄ (58 mg, 0.05 mmol) were used according to the general procedure B to afford compound SET-79A as light yellow solid (75%). ¹H NMR 1H NMR (500 MHz, Chloroform-d) δ 7.59 (d, J = 4.9 Hz, 1H), 7.48 (d, J = 7.7 Hz, 1H), 7.32 - 7.27 (m, 3H), 7.17 (dd, J = 1.7, 7.8 Hz, 2H), 6.98 (d, J = 7.7 Hz, 1H), 6.70 (s, 1H), 3.98 (s, 3H, CH₃), 3.95 - 3.86 (m, 5H, CH₃, CH₂), 3.78 (s, 3H, CH₃). ¹³C NMR (126 MHz, CDCl₃) δ 159.17, 150.67, 147.36, 141.54, 137.60, 135.13, 129.16, 127.72 (2CH), 127.01, 126.74 (2CH), 123.59, 119.18, 113.68, 111.42, 105.47, 55.35, 55.28, 53.33, 46.30.

### General procedure for the deprotection of methoxy groups (H)

To a solution of methoxy containing compounds (1 eq.) cooled to 0°C in DCM (5-10 mL) was added BBr₃ (1M in DCM, 4 eq. per methoxy group) dropwise. Reaction was allowed to warm up to room temperature and stirred overnight. LC/MS analysis revealed complete consumption of the starting material and formation of desired product. Reaction is cooled to 0°C and quenched with water (30 mL) and mixture extracted with diethyl ether (3 × 50 mL). Combined org. phases were dried over Na₂SO₄, evaporated and purified via reverse phase flash chromatography (Hexane/EtOAc, gradient, 0->90% EtOAc).

### Synthesis of N-benzyl-3',4,5-trihydroxy-[1,1'-biphenyl]-2-sulfonamide (SET-69B)

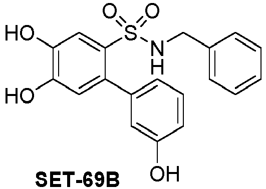

SET-69A (120 mg, 0.29 mmol) and BBr₃ (0.82 mL, 8.7 mmol) were used according to the general procedure H for the deprotection of methoxy groups. Compound SET-69B was obtained as Brown viscous liquid (75%). ¹H NMR (500 MHz, DMSO-*d*₆) δ 9.95 (s, 1H), 9.60 (s, 1H), 9.39 (s, 1H), 7.95 (s, 1H), 7.38 (s, 1H), 7.27 (dd, J = 6.6, 8.1 Hz, 2H), 7.22 (m, 1H), 7.17 (m, 2H), 7.12 (td, J = 2.9, 7.4, 8.1 Hz, 2H), 6.73 (ddd, J = 1.2, 3.4, 5.8 Hz, 2H), 6.60 (s, 1H), 3.78 (d, J = 6.1 Hz, 2H); ¹³C NMR (126 MHz, DMSO-*d*₆) δ 162.80, 156.84, 148.61, 144.51, 141.56, 138.71, 133.25, 129.64, 128.87, 128.63, 127.91, 127.87, 127.50, 120.76, 119.48, 117.05, 116.31, 114.52, 46.41.

### Synthesis of N-benzyl-4,4',5-trihydroxy-[1,1'-biphenyl]-2-sulfonamide (SET-67B)

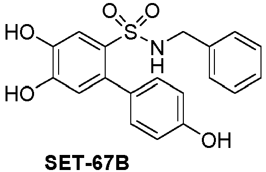

SET-67A (135 mg, 0.31 mmol) and BBr₃ (1.34 mL, 14.1 mmol) were used according to the general procedure H for the deprotection of methoxy groups. Compound SET-67B was obtained as a Brown viscous liquid (72%). ¹H NMR (500 MHz, DMSO-*d*₆) δ 9.89 (s, 1H), 9.55 (s, 1H), 9.43 (s, 1H), 7.95 (s, 2H), 7.39 (s, 1H), 7.27 (m, 2H), 7.22 (m, 1H), 7.15 (m, 2H), 7.12 (m, 2H), 7.00 (t, J = 6.4 Hz, 1H), 6.72 (m, 2H), 6.60 (s, 1H), 3.76 (d, J = 6.3 Hz, 2H) ;¹³C NMR (126 MHz, DMSO-*d*₆) δ 162.80, 156.98, 148.67, 144.20, 138.69, 133.39, 131.01, 130.78, 129.73, 128.60, 127.92, 127.49, 119.96, 116.46, 114.75, 46.36.

### Synthesis of N-benzyl-4,5-dihydroxy-4'-(trifluoromethyl)-[1,1'-biphenyl]-2-sulfonamide (SET-60)

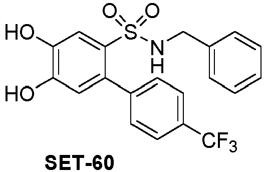

SET-26 (70 mg, 0.12 mmol) and BBr₃ (0.34 mL, 3.6 mmol) were used according to the general procedure H for the deprotection of methoxy groups. Compound SET-60 was obtained as a white solid (70%). ¹H NMR (500 MHz, DMSO-*d*₆) δ 10.06 (s, 1H), 9.76 (s, 1H), 7.67 (m, 3H), 7.50 (d, J = 8.0 Hz, 2H), 7.41 (s, 1H), 7.27 (dd, J = 6.4, 8.0 Hz, 2H), 7.22 (m, 1H), 7.17 (m, 2H), 6.63 (s, 1H), 3.85 (d, J = 6.3 Hz, 2H) ; ¹³C NMR (126 MHz, DMSO-*d*₆) δ 148.82, 145.15, 144.69, 138.59, 131.63, 130.71, 129.75, 128.61, 128.06, 127.91, 127.81, 127.51, 126.01, 124.73, 124.70, 123.84, 119.39, 116.51, 46.28.

### Synthesis of N-benzyl-3',4,4',5-tetrahydroxy-[1,1'-biphenyl]-2-sulfonamide (SET-15)

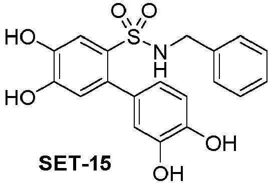

SET-06 (100 mg, 0.225 mmol) and BBr₃ (0.46 mL, 2.7 mmol) were used according to the general procedure for the deprotection of methoxy groups. Compound SET-15 was obtained as a brown liquid (65%). ¹H NMR (500 MHz, DMSO-*d*₆) δ 9.65 (bs, 2H), 8.91 (bs, 2H), 7.38 (d, J = 11.8 Hz, 1H), 7.26 (dd, J = 1.8, 7.7 Hz, 2H), 7.22 (dd, J = 1.5, 7.1 Hz, 1H), 7.17 (m, 2H), 6.80 (m, 1H), 6.74 (m, 1H), 6.68 (m, 1H), 6.60 (s, 1H), 3.75 (d, J = 6.3 Hz, 2H) ; ¹³C NMR (126 MHz, DMSO-*d*₆) δ 157.87, 148.65, 145.07, 144.63, 144.15, 134.90, 134.88, 133.51, 131.20, 129.96, 129.89, 129.44, 120.96, 119.83, 117.63, 116.36, 115.42, 115.25, 45.68.

### Synthesis of N-benzyl-3',4,5,5'-tetrahydroxy-[1,1'-biphenyl]-2-sulfonamide (SET-71B)

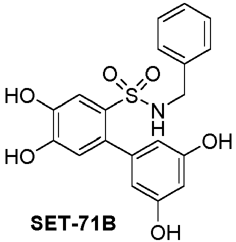

SET-71A (100 mg, 0.22 mmol) and BBr₃ (1.28 mL, 13.53 mmol) were used according to the general procedure H for the deprotection of methoxy groups. Compound SET-71B was obtained as a brown liquid (52%). ¹H NMR (500 MHz, DMSO-*d₆*) δ 9.94 (s, 1H), 9.56 (s, 1H), 9.23 (s, 2H), 7.63 (t, J = 6.3 Hz, 1H), 7.44 (s, 1H), 7.27 (m, 2H), 7.20 (m, 3H), 6.84 (s, 1H), 6.55 (d, J = 2.3 Hz, 2H), 6.49 (t, J = 2.3 Hz, 1H), 3.89 (d, J = 6.3 Hz, 2H). ¹³C NMR (126 MHz, DMSO-*d₆*) δ 157.87, 148.60, 144.41, 141.90, 134.88, 133.46, 129.95, 129.89, 129.33, 119.21, 116.23, 115.44, 115.27, 108.41,101.92, 45-77.

### Synthesis of N benzyl-4,5-dihydroxy-3',5'-bis(trifluoromethyl)-[1.1'-biphenyll-2-sulfonamide (SET-74B)

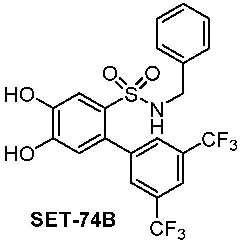

SET-74A (100 mg, 0.19 mmol) and BBr₃ (0.72 mL, 7.6 mmol) were used according to the general procedure H for the deprotection of methoxy groups. Compound SET-74B was obtained as a brown liquid (65%). ¹H NMR (500 MHz, Methanol-*d*₄) δ 7.89 (s, 1H), 7.75 (d, J = 1.7 Hz, 2H), 7.55 (s, 1H), 7.21 (dd, J = 1.7, 5.7 Hz, 3H), 7.08 - 7.00 (m, 2H), 6.63 (s, 1H), 3.91 (s, 2H). ¹³C NMR (126 MHz, MeOD) δ 173.02, 164.85, 150.23, 146.48, 143.37, 138.58, 131.78, 131.60, 130.94, 129.38 (2CH), 129.02 (2CH), 128.44, 125.95, 123.79, 122.09, 120.01, 117.90, 61.54, 47.49.

### Synthesis of N-benzyl-4,5-dihydroxy-4'-(trifluoromethoxy)-[1,1'-biphenyl]-2-sulfonamide (SET-75B)

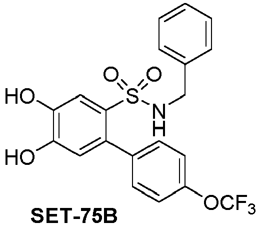

SET-75A (100 mg, 0.21 mmol) and BBr₃ (0.59 mL, 6.3 mmol) were used according to the general procedure H for the deprotection of methoxy groups. Compound SET-75B was obtained as a brown liquid (72%). ¹H NMR (500 MHz, Methanol-*d*₄) δ 7.50 (s, 1H), 7.38 - 7.32 (m, 2H), 7.27 - 7.16 (m, 5H), 7.12 - 7.08 (m, 2H), 6.65 (s, 1H), 3.87 (s, 2H). ¹³C NMR (126 MHz, MeOD) δ 150.01, 149.85, 145.76, 140.19, 138.80, 133.63, 132.61 (2CH), 130.56, 129.37 (2CH), 128.95 (2CH), 128.44, 122.96, 121.05, 120.22, 117.62, 61.56, 47.55.

*Synthesis of N-benzyl-3'-fluoro-4,4',5-trihydroxy-[1,1'-biphenyl]-2-sulfonamide (SET-77B)*

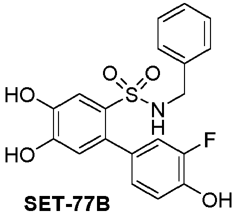

SET-77A (50 mg, 0.11 mmol) and BBr₃ (0.55 mL, 5.8 mmol) were used according to the general procedure H for the deprotection of methoxy groups. Compound SET-77B was obtained as a light yellow viscous liquid (65%). ¹H NMR (500 MHz, Methanol-*d₄*) δ 7.49 (s, 1H), 7.29 - 7.16 (m, 3H), 7.09 (dd, J = 1.8, 7.7 Hz, 2H), 7.00 (dd, J = 2.1, 12.1 Hz, 1H), 6.94 - 6.80 (m, 2H), 6.65 (s, 1H), 3.84 (s, 2H). ¹³C NMR (126 MHz, MeOD) δ 152.64, 150.72, 149.80, 145.61, 145.28, 138.46, 133.71, 132.21, 130.16, 129.20, 128.85, 128.28, 126.87, 120.23, 118.59, 117.71, 117.68, 117.38, 47.52.

### Synthesis of N-benzyl-4,4 ',5-trihydroxy-3'-(trifluoromethyl)-[1,1'-biphenyl]-2-sulfonamide (SET-78B)

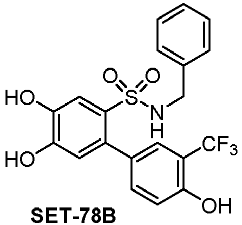

SET-78A (100 mg, 0.20 mmol) and BBr₃ (0.85 mL, 9.0 mmol) were used according to the general procedure H for the deprotection of methoxy groups. Compound SET-78B was obtained as a light brown viscous liquid (63%). ¹H NMR (500 MHz, Methanol-*d₄*) δ 7.51 (s, 1H), 7.48 - 7.43 (m, 2H), 7.21 (td, J = 2.7, 5.8 Hz, 3H), 7.13 - 7.03 (m, 3H), 6.63 (s, 1H), 3.86 (s, 2H). ¹³C NMR (126 MHz, MeOD) δ 158.31, 150.07, 145.67, 138.63, 136.21, 133.50, 132.73, 130.65, 129.37 (2CH), 128.99 (2CH), 128.44, 126.18, 120.41, 118.73, 118.49, 117.72, 112.52, 47.61.

### Synthesis of N-benzyl-2-(6-chloro-2-methoxypyridin-'3-yl)-4,5-dihydroxybenzenesulfonamide (SET-79B)

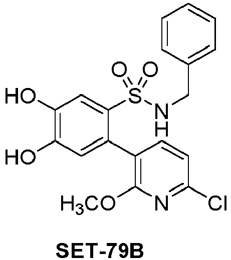

SET-79A (25 mg, 0.05 mmol) and BBr₃ (0.27 mL, 2.8 mmol) were used according to the general procedure H for the deprotection of methoxy groups. Compound SET-79B was obtained as a brown liquid (65%). ¹H NMR (500 MHz, Methanol-*d*₄) δ 7.98 (s, 2H, 2OH), 7.44 (s, 1H), 7.38 (d, J = 7.6 Hz, 1H), 7.31- 7.20 (m, 3H), 7.20 - 7.13 (m, 2H), 6.95 (d, J = 7.6 Hz, 1H), 6.60 (s, 1H), 3.92 (d, J = 9.3 Hz, 2H, CH₂), 3.79 (s, 3H, OCH₃). ¹³C NMR (126 MHz, MeOD) δ 162.18, 150.02, 148.32, 145.98, 142.95, 138.79, 130.66, 129.24 (2CH), 128.72 (2CH), 128.32, 128.25, 122.77, 119-73, 117-39, 116-53, 54-34, 47-38.

### General Procedure for the Synthesis of 2-bromo-4,5-dimethoxybenzenesulfonyl chloride (2)

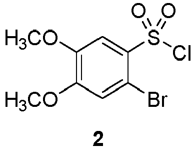

4-Bromoveratrole 1 (4.0 ml, 27.8 mmol) was added in a dropwise over 20 min. to a well stirred flask containing chlorosulfonic acid (8 ml) cooled to 0 C. The black mixture was then stirred for a further 30 min before cautious addition to ice (150 ml). After melting DCM (100ml) was added and the phase were separated. The aqueous phase was extracted with DCM (100ml) and the combination organic phases were dried over MgSO₄. Filtration and solvent removal under reduced pressure gave the sulfonyl chloride 2 (7.17 g 82%) as a white solid which was further purified by recrystallisation from diethylether. m.p. 78 C ; ¹H-NMR (400 MHz, CDCl₃) δ 3.94 (s, 3H), 3.99 (s, 3H), 7.18 (s, 1H), 7.59 (s, 1H) ; ¹³C-NMR (100 MHz, CDCl₃) δ 56.5, 56.7, 112.5, 113.0, 117.7, 134.6, 147.8, 154.1.

### Synthesis of 2-bromo-N-(4-fluorobenzyl)-4,5-dimethoxybenzenesulfonamide (SET-11)

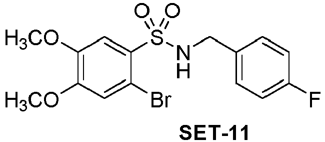

2 (10.0 g, 31.69 mmol) was added to a solution of fluorobenzylamine (4.15 mL, 38.03 mmol) and DIPEA (16.45 mL, 95.07 mmol) in DCM (30 mL) cooled to 0°C. Reaction was allowed to warm to r.t. and stirred for 6 hour, until complete conversion of the starting material has been confirmed by TLC (3% EtOAc in DCM). Reaction is quenched with water (30 mL). Crude product was extracted with DCM (3 × 30 mL). Combined org. phases were washed with 1M HCl (3 × 50 mL), dried over Na₂SO₄ and evaporated to obtain the yellow oil, which was recrystallized from hot MeOH to provide compound SET-11 as a white crystalline solid (10.41 g, 82% ). ¹H NMR (400 MHz, DMSO-d6) δ 8.24 (s, 1H, NH), 7.38 (s, 1H, Ar), 7.26 (s, 1H, Ar), 7.25 - 7.15 (m, 5H, Ar), 4.08 (s, 2H, CH₂), 3.84 (s, 3H, CH3), 3.76 (s, 3H, CH3); ¹³C NMR (101 MHz, DMSO-d6) δ 151.7, 147.3, 137.6, 131.5, 128.0, 127.6, 127.0, 117.4, 113.3, 110.6, 56.3, 55.8, 46.1.

### General procedure for the synthesis of biaryl sulfonamides (Cross coupling)

Biaryl sulfonamides were prepared according to the slightly modified described procedure.¹ Compound SET-11 (1.0 eq.), appropriate boronic acid (1.5 eq.), potassium carbonate (4.0 eq.) and Pd(PPh₃)₄ (0.03 eq.) were suspended in the mixture of toluene:ethanol:water/5:2:1 (13 mL). Sequential applying of N₂ flow and vacuum were used to degas the mixture before heating in a MW reactor at 120 °C for 1h. The crude product was extracted with ethyl acetate (3 × 30 mL). To the organic phase was dried over sodium sulfate. The mixture was then filtered through the bed of celite and solvent removed under reduced pressure. The residue was purified by flash chromatography using Hexane/EtOAc (gradient) to afford the biaryl sulfonamides.

### Synthesis of N-(4-fluorobenzyl)-3',4,5-trimethoxy-[1,1'-biphenyl]-2-sulfonamide (SET-68A)

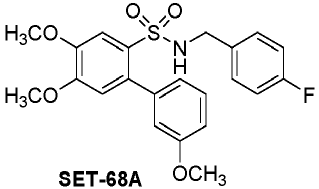

SET-11 (400 mg, 0.98 mmol), 3-Methoxybenzeneboronic acid (224 mg, 1.48 mmol), K₂CO₃ (540 mg, 3.92 mmol) and Pd(PPh₃)₄ (23 mg, 0.02 mmol) were used according to the general procedure B to afford compound SET-68A as white solid (67%). ¹H NMR (400 MHz, DMSO-*d*₆) δ ¹H NMR (500 MHz, DMSO-*d*₆) δ 7.65 (t, *J* = 6.3 Hz, 1H), 7.44 (s, 1H), 7.27 (m, 3H), 7.21 (dd, J = 5.7, 8.6 Hz, 2H), 7.09 (t, J = 8.9 Hz, 2H), 6.82 (s, 1H), 5.75 (s, 1H), 3.87 (d, *J* = 6.2 Hz, 2H), 3.82 (d, *J* = 7.5 Hz, 6H), 3.75 (s, 3H); ¹³C NMR (126 MHz, DMSO-*d*₆) δ 158.80, 151.05, 147.79, 141.46, 138.40, 134.50, 131.13, 129.00, 128.64, 128.07, 127.56, 122.37, 115.79, 115.55, 113.27, 111.76, 56.36, 56.33, 55.43, 46.51.

### Synthesis of N-(4-fluorobenzyl)-4,4',5-trimethoxy-[1.1'-biphenyl]-2-sulfonamide (SET-66A)

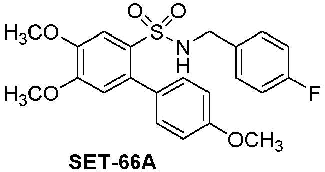

SET-11 (300 mg, 0.58 mmol), 4-Methoxybenzeneboronic acid (170 mg, 1.16 mmol), K₂CO₃ (400 mg, 2.9 mmol) and Pd(PPh₃)₄ (34 mg, 0.02 mmol) were used according to the general procedure B to afford compound SET-66A as a white solid (83%). ¹H NMR (500 MHz, DMSO-*d*₆) δ 7.60 (t, J = 6.3 Hz, 1H), 7.41 (s, 1H), 7.30 (m, 2H), 7.21 (dd, J = 5.7, 8.6 Hz, 2H), 7.09 (t, J = 8.9 Hz, 2H), 6.93 (d, J = 8.7 Hz, 2H), 6.78 (s, 1H), 3.84 (d, J = 6.4 Hz, 2H), 3.82 (d, J = 3.7 Hz, 6H), 3.79 (s, 3H); ¹³C NMR (126 MHz, DMSO-*d*₆) δ 162.72, 160.79, 159.05, 151.09, 147.54, 134.56, 132.31, 131.12, 130.09, 130.03, 115.87, 115.42, 115.25, 113.41, 111.84, 56.31, 55.56, 45.70.

### Synthesis of N-[4-fluorobenzyl)-4,5-dimethoxy-4'-(trifluoromethyl)-[1,1'-biphenyl]-2-sulfonamide (SET-25)

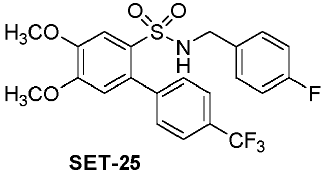

SET-11 (500 mg, 0.96 mmol), 4-Trifluoromethylbenzeneboronic acid (270 mg, 1.44 mmol), K₂CO₃ (530 mg, 3.84 mmol) and Pd(PPh₃)₄ (340 mg, 0.03 mmol) were used according to the general procedure B to afford compound SET-25 as white solid (75%). ¹H NMR (500 MHz, DMSO- *d*₆) δ 7.93 (t, *J* = 6.3 Hz, 1H), 7.73 (d, *J* = 8.1 Hz, 2H), 7.57 (d, *J* = 8.0 Hz, 2H), 7.45 (s, 1H), 7.21 (dd, J = 5.7, 8.6 Hz, 2H), 7.09 (t, J = 8.9 Hz, 2H), 6.86 (s, 1H), 3.92 (d, *J* = 6.2 Hz, 2H), 3.82 (d, *J* = 1.9 Hz, 6H) ; ¹³C NMR (126 MHz, DMSO-*d*₆) δ 160.09, 153.05, 151.09, 147.54, 134.56, 132.31, 131.12, 130.09, 130.03, 115.87, 115.42, 115.25, 113.41, 111.84, 56.31, 55.56, 46.20.

### Synthesis of N-(4-fluorobenzyl)-'3',4,4',5-tetramethoxy-[1,1'-biphenyl]-2-sulfonamide (SET-12)

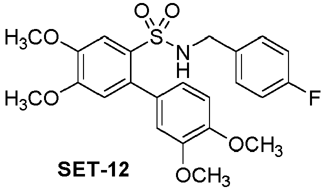

SET-11 (500 mg, 1.23 mmol), 3,4-Dimethoxybenzeneboronic acid (330 mg, 1.84 mmol), K₂CO₃ (670 mg, 4.92 mmol) and Pd(PPh₃)₄ (30 mg, 0.03 mmol) were used according to the general procedure B to afford compound SET-12 as white solid (82%). ¹H NMR (500 MHz, DMSO-*d₆*) δ 7.50 (t, J = 6.3 Hz, 1H, NH), 7.46 (s, 1H, Ar), 7.21 (dd, J = 5.7, 8.6 Hz, 2H), 7.14 (t, J = 8.9 Hz, 2H), 7.01 (d, J = 2.0 Hz, 1H, Ar), 6.96 (d, J = 8.3 Hz, 1H, Ar), 6.92 (dd, J = 8.2, 2.0 Hz, 1H, Ar), 6.84 (s, 1H, Ar), 3.86 (d, J = 6.2 Hz, 2H, CH₂), 3.83 (s, 3H, CH₃), 3.81 (s, 3H, CH₃), 3.78 (s, 3H, CH₃), 3.73 (s, 3H, CH₃); ¹³C NMR (101 MHz, DMSO-*d₆*) δ 160.09, 150.6, 148.2, 147.6, 147.1, 137.9, 134.2, 132.0, 130.6, 128.2, 127.6, 127.1, 121.8, 115.4, 113.7, 111.5, 111.0, 55.9, 55.9, 55.5, 55.4, 46.0.

### Synthesis of N-(4-fluorobenzyl)-3',4,5,5'-tetramethoxy-[1,1'-biphenyl]-2-sulfonamide (SET-70A)

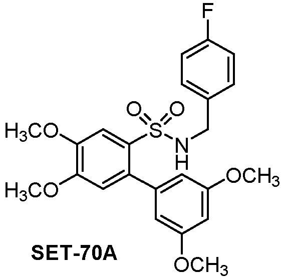

SET-11 (500 g, 0.96 mmol), 3,5-Dimethoxybenzeneboronic acid (340 mg, 0.93 mmol), K₂CO₃ (660 mg, 4.8 mmol) and Pd(PPh₃)₄ (40 mg, 0.03 mmol) were used according to the general procedure B to afford compound SET-70A as white solid (67%). ¹H NMR (500 MHz, DMSO-*d₆*) δ 7.65 (t, J = 6.3 Hz, 1H), 7.42 (s, 1H), 7.22 (dd, J = 5.7, 8.6 Hz, 2H), 7.09 (t, J = 8.9 Hz, 2H), 6.83 (s, 1H), 6.53 (d, J = 2.3 Hz, 2H), 6.48 (t, J = 2.3 Hz, 1H), 3.87 (d, J = 6.0 Hz, 2H), 3.82 (d, J = 6.3 Hz, 6H), 3.74 (s, 6H); ¹³C NMR (126 MHz, DMSO-*d₆*) δ 161.66, 160.51, 159.73, 158.88, 158.52, 150.00, 146.72, 140.81, 133.55, 133.41, 129.86, 129.02, 128.95, 114.35, 114.19, 110.69, 107.32, 98.59, 93.31, 90.85, 78.57, 55-30, 55.26, 54.51, 54.29, 44.69.

### Synthesis of N-(4-fluorobenzyl)-4,5-dimethoxy-3',5'-bis(trifluoromethyl)-[1,1'-biphenyl]-2-sulfon-amide (SET-73A)

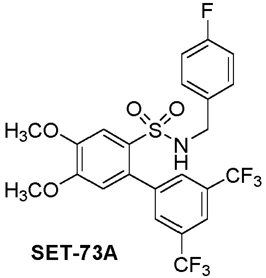

SET-11 (700 g, 1.72 mmol), 3,5-Ditrifluoromethylbenzeneboronic acid (880 mg, 3.44 mmol), K₂CO₃ (950 mg, 6.8 mmol) and Pd(PPh₃)₄ (92 mg, 0.08 mmol) were used according to the general procedure B to afford compound SET-73A as white solid (88%). ¹H NMR (500 MHz, Chloroform-*d*) δ 7.87 (s, 1H), 7.75 (d, J = 1.6 Hz, 2H), 7.63 (s, 1H), 6.99 (dd, J = 5.4, 8.5 Hz, 2H), 6.92 (t, J = 8.6 Hz, 2H), 6.68 (s, 1H), 3.99 (s, 3H), 3.93 (s, 3H), 3.89 (d, J = 3.5 Hz, 2H); ¹³C NMR (126 MHz, CDCl₃) δ 163.86, 161.90, 152.26, 149.01, 141.24, 131.93, 131.68, 131.61, 131.41, 130.69, 130.41, 130.16, 130.09, 124.55, 122.43, 116.19, 116.02, 114.80, 113.03, 77.16, 56.91, 56.88, 46.83.

### General procedure for the deprotection of methoxy groups (H)

To a solution of methoxy containing compounds (1 eq.) cooled to 0°C in DCM (5-10 mL) was added BBr₃ (1M in DCM, 4 eq. per methoxy group) dropwise. Reaction was allowed to warm up to room temperature and stirred overnight. LC/MS analysis revealed complete consumption of the starting material and formation of the desired product. Reaction is cooled to 0 °C and quenched with water (30 mL) and the mixture was extracted with diethyl ether (3 × 50 mL). Combined organic phases were dried over Na₂SO₄, evaporated and purified via reverse phase flash chromatography (Hexane/EtOAc, gradient, 0->90% EtOAc).

### Synthesis of N-(4-fluorobenzyl)-3',4,5-trihydroxy-[1,1'-biphenyl]-2-sulfonamide (SET-68B)

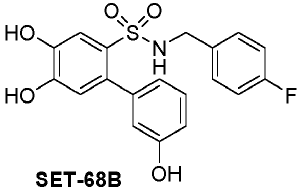

SET-68A (100 mg, 0.23 mmol) and BBr₃ (0.66 mL, 6.95 mmol) were used according to the general procedure H for the deprotection of methoxy groups. Compound SET-68B was obtained as a brown viscous liquid (52%). ¹H NMR (500 MHz, DMSO-*d₆*) δ 9.96 (s, 1H), 9.60 (s, 1H), 9.39 (s, 1H), 7.95 (s, 1H), 7.37 (s, 1H), 7.23 - 7.16 (m, 3H), 7.15 - 7.07 (m, 3H), 6.75 - 6.71 (m, 2H), 6.61 (s, 1H), 3.78 (d, J = 6.1 Hz, 2H); ¹³C NMR (126 MHz, DMSO-*d₆*) δ 162.80, 162.70, 160.77, 156.83, 148.64, 144.50, 141.54, 134.96, 134.93, 133.27, 129.92, 129.86, 129.56, 128.87, 120.75, 119.49, 117.05, 116.34, 115.42, 115.25, 114.53, 45.65.

### Synthesis of N-(4-fluorobenzyl)-4,4',5-trihydroxy-[1,1'-biphenyl]-2-sulfonamide (SET-66B)

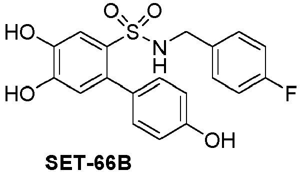

SET-66A (60 mg, 0.14 mmol) and BBr₃ (0.36 mL, 2.08 mmol) were used according to the general procedure H for the deprotection of methoxy groups. Compound SET-66B was obtained as a Brown viscous liquid (65%). ¹H NMR (500 MHz, DMSO-d₆) 9.88 (s, 1H), 9.53 (s, 1H), 9.41 (s, 1H), 7.95 (s, 1H), 7.36 (s, 1H), 7.18 (m, 2H), 7.10 (m, 4H), 6.71 (d, J = 8.5 Hz, 2H), 6.59 (s, 1H), 3.75 (d, J = 6.3 Hz, 2H); ¹³C NMR (126 MHz, DMSO-*d₆*) δ 156.97, 148.68, 144.19, 134.96, 134.94, 133.40, 130.99, 130.76, 129.93, 129.87, 129.65, 119-97, 116.48, 115.39, 115.22, 114.75, 45.58.

### Synthesis of N-(4-fluorobenzyl)-4,5-dihydroxy-4'-(trifluoromethyl)-[1,1'-biphenyl]-2-sulfonamide (SET-57)

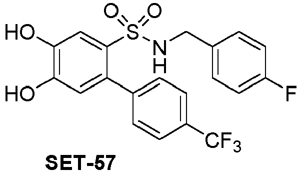

SET-25 (190 mg, 0.40 mmol) and BBr₃ (0.76 mL, 8.1 mmol) were used according to the general procedure H for the deprotection of methoxy groups. Compound SET-57 was obtained as a white solid (72%). ¹H NMR (500 MHz, DMSO-*d₆*) δ 10.07 (s, 1H), 9.76 (s, 1H), 7.68 (d, J = 8.2 Hz, 2H), 7.50 (d, J = 8.0 Hz, 2H), 7.39 (s, 1H), 7.20 (dd, J = 5.7, 8.6 Hz, 2H), 7.09 (m, 2H), 6.63 (s, 1H), 3.84 (d, J = 6.1 Hz, 2H); ¹³C NMR (126 MHz, DMSO-*d₆*) δ 160.78, 148.86, 145.15, 144.65, 134.82, 131.64, 130.69, 129.93, 129.86, 129.65, 128.08, 127.83, 126.00, 124.77, 124.74, 124.71, 124.68, 123.84, 119.39, 116.52, 115.40, 115.23, 45.52.

### Synthesis of N-(4-fluorobenzyl)-3',4,4',5-tetrahydroxy-[1,1'-biphenyl]-2-sulfonamide (SET-13)

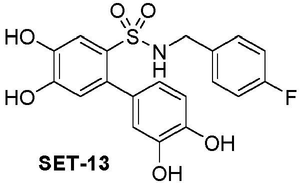

SET-12 (215 mg, 0.45 mmol) and BBr₃ (0.86 mL, 9.0 mmol) were used according to the general procedure for the deprotection of methoxy groups. Compound SET-13 was obtained as a brown liquid (68%). ¹H NMR (500 MHz, DMSO-d₆) δ 9.65 (s, 2H), 8.91 (s, 2H), 7.35 (s, 1H), 7.19 (dd, J = 5.7, 8.6 Hz, 2H), 7.09 (t, J = 8.9 Hz, 2H), 6.73 (d, J = 2.1 Hz, 1H), 6.68 (d, J = 8.1 Hz, 1H), 6.59 (m, 2H), 3.74 (s, 2H); ¹³C NMR (126 MHz, DMSO-*d₆*) δ 162.69, 160.76, 148.65, 145.07, 144.63, 144.15, 134.90, 134.88, 133.51, 131.20, 129.96, 129.89, 129.44, 120.96, 119.83, 117.63, 116.36, 115.42, 115.25, 115.21, 45.68.

### Synthesis of N-(4-fluorobenzyl)-3',4,5,5'-tetrahydroxy-[1,1'-biphenyl]-2-sulfonamide (SET-70B)

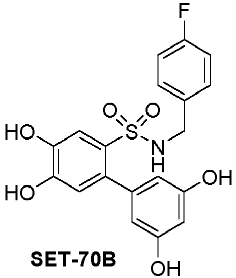

SET-70A (100 mg, 0.21 mmol) and BBr₃ (1.23 mL, 13.0 mmol) were used according to the general procedure H for the deprotection of methoxy groups. Compound SET-70B was obtained as a brown liquid (61%). ¹H NMR (500 MHz, DMSO-*d₆*) δ 9.94 (s, 1H), 9.56 (s, 1H), 9.23 (s, 2H), 7.34 (s, 1H), 7.22 (dd, J = 5.7, 8.6 Hz, 2H), 7.10 (m, 2H), 6.97 (t, J = 6.4 Hz, 1H), 6.60 (s, 1H), 6.20 (d, J = 2.2 Hz, 2H), 6.16 (t, J = 2.2 Hz, 1H), 3.78 (d, J = 6.4 Hz, 2H); ¹³C NMR (126 MHz, DMSO-*d₆*) δ 160.78, 157.87, 148.60, 144.41, 141.90, 134.88, 133.46, 129.95, 129.89, 129.33, 119.21, 116.23, 115.44, 115.27, 108.41, 101.92, 45.77.

### Synthesis of N-(4-fluorobenzyl)-4,5-dihydroxy-3',5'-bis(trifluoromethyl)-[1,1'-biphenyl]-2-sulfon-amide (SET-73B)

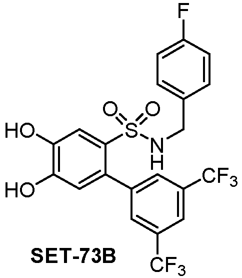

SET-73A (45 mg, 0.08 mmol) and BBr₃ (0.30 mL, 3.2 mmol) were used according to the general procedure H for the deprotection of methoxy groups. Compound SET-73B was obtained as a brown liquid (70%). ¹H NMR (500 MHz, Methanol-*d₄*) δ 7.98 (s, 1H), 7.90 (d, J = 1.7 Hz, 1H), 7.78 (d, J = 1.6 Hz, 1H), 7.52 (s, 1H), 7.07 (dd, J = 5.4, 8.5 Hz, 2H), 6.95 (t, J = 8.8 Hz, 2H), 6.65 (s, 1H), 3.89 (s, 2H).

### Synthesis of N-benzyl-2-bromo-4,5-dimethoxybenzenesulfonamide (SET-04)

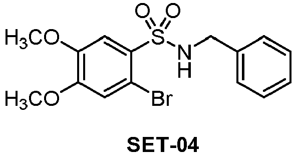

2 (10.0 g, 31.69 mmol) was added to a solution of benzylamine (4.15 mL, 38.03 mmol) and DIPEA (16.45 mL, 95.07 mmol) in DCM (30 mL) cooled to 0°C. Reaction was allowed to warm to r.t. and stirred for one hour, until complete conversion of the starting material has been confirmed by TLC (3% EtOAc in DCM). Reaction is quenched with water (30 mL). Crude product was extracted with DCM (3 × 30 mL). Combined organic Phases were washed with 1M HCl (3 × 50 mL), dried over Na₂SO₄ and evaporated to obtain the white solid SET-04 (10.7 g, 90%). ¹H NMR (400 MHz, DMSO-*d₆*) δ 8.24 (s, 1H, NH), 7.38 (s, 1H, Ar), 7.26 (s, 1H, Ar), 7.25 - 7.15 (m, 5H, Ar), 4.08 (s, 2H, CH₂), 3.84 (s, 3H, CH₃), 3.76 (s, 3H, CH₃); ¹³C NMR (101 MHz, DMSO- *d₆*) δ 151.7, 147.3, 137.6, 131.5, 128.0, 127.6, 127.0, 117.4, 113.3, 110.6, 56.3, 55.8, 46.1.

### General procedure for the synthesis of biaryl sulfonamides (Cross coupling)

Biaryl sulfonamides were prepared according to the slightly modified described procedure.¹ Compound SET-04 (1.0 eq.), appropriate boronic acid (1.5 eq.), potassium carbonate (4.0 eq.) and Pd(PPh₃)₄ (0.03 eq.) were suspended in the mixture of toluene:ethanol:water/5:2:1 (13 mL). Sequential applying of N₂ flow and vacuum were used to degas the mixture before heating in a MW reactor at 120 °C for 1h. The crude product was extracted with ethyl acetate (3 × 30 mL). To the organic phase was dried over sodium sulfate. The mixture was then filtered through the bed of celite and solvent removed under reduced pressure. The residue was purified by flash chromatography using Hexane/EtOAc (gradient) to afford the biaryl sulfonamides.

### General procedure for the synthesis of sultams via cyclization of secondary sulfonamides (C)

Cyclization was achieved according to the procedure described in the literatures appropriate sulfonamide (1 eq.), PIDA (1.10 eq.), I₂ (1.10 eq.) and K₂CO₃ (1.50 eq.) were suspended in DCM (20 mL). The dark red solution was stirred at 35 °C for 1-3h, until almost complete conversion was determined by TLC (3%EtOAc in DCM). Reaction is quenched with sat. aq. Na₂S₂O₅, and mixture was stirred at the r.t. until discoloration of the solution. Crude product was extracted with DCM and purified via flash chromatography (DCM/EtOAc, gradient, 0->1% EtOAc) and triturated with MeOH to afford sultams.

*Synthesis of 6-benzyl-2,3,8-trimethoxy-6H-dibenzo[c,e][1,2]thiazine 5,5-dioxide (SET-67C)*

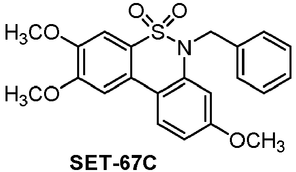

SET-67A (180 mg, 0.43 mmol), PIDA (150 mg, 0.47 mmol), I₂ (110 mg, 0.47 mmol) and K₂CO₃ (89 mg, 0.65 mmol) were used according to the general procedure C to afford compound SET-67C as a white solid (75%). ¹H NMR (500 MHz, DMSO-*d₆*) δ 8.11 (d, J = 9.6 Hz, 1H), 7.49 (s, 1H), 7.37 (s, 1H), 7.18 (m, 5H), 6.90 (m, 2H), 5.16 (s, 2H), 3.95 (s, 6H), 3.75 (s, 3H); ¹³C NMR (126 MHz, DMSO-*d₆*) δ 159.30, 151.66, 147.73, 137.78, 135.56, 127.83, 126.86, 126.67, 126.39, 124.99, 124.92, 116.62, 110.43, 107.22, 105.30, 103.23, 55.55, 55.42, 54.88, 48.77.

### Synthesis of 6-benzyl-2,3,9-trimethoxy-6H-dibenzo[c,e][1,2]thiazine 5,5-dioxide (SET-69C)

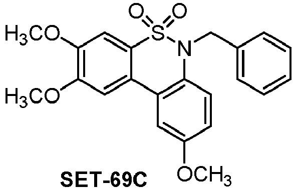

SET-69A (180 mg, 0.43 mmol), PIDA (150 mg, 0.47 mmol), I₂ (110 mg, 0.47 mmol) and K₂CO₃ (89 mg, 0.65 mmol) were used according to the general procedure C to afford compound SET-69C as mixture of two positional isomers a white solid, purified by flash chromatography using (DCM/EtOAc, gradient, o->i% EtOAc) to afford (45% SET-69C) and 35% other Isomer. ¹H NMR (500 MHz, DMSO-*d₆*) δ 7.62 (d, J = 2.9 Hz, 1H), 7.37 (m, 2H), 7.29 (d, J = 8.9 Hz, 1H), 7.26 (s, 1H), 7.20 (s, 1H), 7.06 (m, 1H), 6.99 (td, J = 2.1, 8.7 Hz, 2H), 6.69 (dt, J = 1.3, 7.0 Hz, 1H), 4.96 (s, 2H), 3.95 (d, J = 4.3 Hz, 6H), 3.89 (s, 3H); ¹³C NMR (126 MHz, DMSO-d₆) δ 156.14, 153.29, 148.57, 148.46, 135.21, 130.06, 127.85, 127.29, 126.86, 126.68, 125.32, 124.64, 116.50, 111.30, 109.85, 108.26, 108.18, 104.23, 55.67, 55.53, 55.02, 52.70.

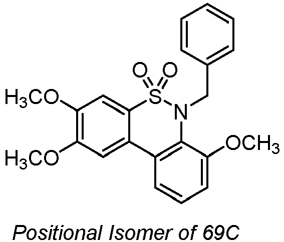

Yield: 35%; ¹H NMR (500 MHz, DMSO-*d₆*) δ 7.57 (dd, J = 1.2, 8.2 Hz, 1H), 7.51 (s, 1H), 7.26 (s, 1H), 7.20 (s, 1H), 7.17 (m, 2H), 7.06 (m, 1H), 6.90 (dd, J = 6.9, 8.3 Hz, 2H), 6.69 (dt, J = 1.3, 7.0 Hz, 1H), 4.77 (s, 2H), 3.89 (s, 3H), 3.84 (d, J = 1.6 Hz, 6H); ¹³C NMR (126 MHz, DMSO-*d₆*) δ 151.45, 151.21, 148.57, 148.46, 133.29, 128.38, 127.65, 126.94, 126.76, 125.72, 125.20, 123.06, 114.58, 111.30, 109.85, 108.26, 108.18, 103.66, 55.47, 55.35, 55.02, 51.11.

### Synthesis of 6-benzyl-2,2-dimethoxy-8-(trifluoromethyl)-6H-dibenzo[c,e][1,2]thiazine 5,5-dioxide (SET-61)

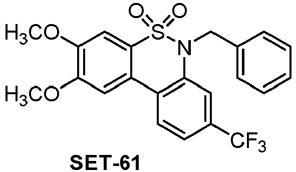

SET-26 (100 mg, 0.22 mmol), PIDA (77 mg, 0.24 mmol), I₂ (60 mg, 0.24 mmol) and K₂CO₃ (46 mg, 0.33 mmol) were used according to the general procedure C to afford compound SET-61 as a white solid (72%). ¹H NMR (500 MHz, DMSO-*d₆*) δ 8.44 (d, J = 8.3 Hz, 1H), 7.77 (d, J = 1.7 Hz, 1H), 7.65 (s, 1H), 7.47 (s, 1H), 7.19 (m, 4H), 7.10 (m, 2H), 5.26 (s, 2H), 3.97 (d, J = 7.0 Hz, 6H); ¹³C NMR (126 MHz, DMSO-*d₆*) δ 152.76, 150.49, 137.82, 137.59, 135.96, 131.15, 130.79, 128.97, 128.63, 128.53, 128.18, 128.16, 128.12, 128.09, 127.80, 127.40, 124.31, 121.62, 121.58, 118.73, 109.59, 104.48, 56.81, 56.71, 50.36, 19.03.

### Synthesis of 6-benzyl-2,3,8,9-tetramethoxy-6H-dibenzo[c,e][1,2]thiazine 5,5-dioxide (SET-08)

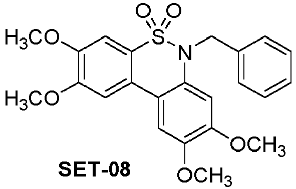

SET-06 (213 mg, 0.48 mmol), PIDA (170.16 mg, 0.52 mmol), I₂ (134 mg, 0.53 mmol) and K₂CO₃ (99.5 mg, 0.72 mmol) were used according to the general procedure C to afford compound SET-08 as a white solid (55%). ¹H NMR (500 MHz, Chloroform-d) δ 7.40 (s, 1H, Ar), 7.22-7.16 (m, 6H, Ar), 7.08 (s, 1H, Ar), 6.54 (s, 1H, Ar), 4.79 (s, 2H, CH₂), 4.01 (d, J = 8.3 Hz, 6H, CH₃), 3.95 (s, 3H, CH₃), 3.67 (s, 3H, CH₃); ¹³C NMR (126 MHz, CDCl₃) δ 152.20, 149.81, 148.72, 147.37, 135.52, 132.03, 131.95, 128.44, 128.32, 127.73, 126.56, 126.32, 118.73, 107.40, 107.19, 106.99, 105.32, 77.16, 56.37, 56.34, 56.25, 55.74, 54.43.

### Synthesis of 6-benzyl-2,3,7,9-tetramethoxy-6H-dibenzo[c,e][1,2]thiazine 5,5-dioxide (SET-71C)

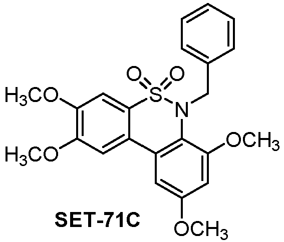

SET-71A (150 mg, 0.33 mmol), PIDA (115 mg, 0.36 mmol), I₂ (91 mg, 0.36 mmol) and K₂CO₃ (67 mg, 0.49 mmol) were used according to the general procedure C to afford compound SET-71C as a white solid (69%). ¹H NMR (500 MHz, DMSO-*d₆*) δ 7.21 (s, 1H), 7.18 (s, 1H), 7.03-6.92 (m, 5H, Ar), 6.75 (s, 1H), 6.52 (s, 1H), 4.64 (s, 2H), 3.94 (s, 3H), 3.89 (s, 3H), 3.88 (s, 3H), 3.86 (s, 3H); ¹³C NMR (126 MHz, DMSO-*d₆*) δ 152.20, 149.81, 148.72, 147.37, 135.52, 132.03, 131.95, 128.44, 128.32, 127.73, 126.56, 126.32, 118.73, 107.40, 107.19, 106.99, 105.32, 77.16, 56.37, 56.34, 56.25, 55.74, 54.43.

### Synthesis of 6-benzyl-2,3-dimethoxy-8-(trifluoromethoxy)-6H-dibenzo[c,e][1,2]thiazine 5,5-dioxide (SET-75C)

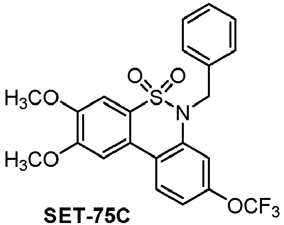

SET-75A (210 mg, 0.44 mmol), PIDA (150 mg, 0.48 mmol), I₂ (121 mg, 0.48 mmol) and K₂CO₃ (91 mg, 0.66 mmol) were used according to the general procedure C to afford compound SET-75C as a white solid (70%). ¹H NMR (500 MHz, Chloroform-d) δ 7.85 (d, J = 8.8 Hz, 1H, Ar), 7.45 (s, 1H, Ar), 7.24 - 7.17 (m, 6H, Ar), 7.10 (ddd, J = 1.1, 2.4, 8.7 Hz, 1H, Ar), 7.03 (dd, J = 1.1, 2.3 Hz, 1H, Ar), 5.05 (s, 2H, CH₂), 4.01 (d, J = 4.1 Hz, 6H, 2OCH₃). ¹³C NMR (126 MHz, CDCl₃) δ 152.61,149.79, 149.33, 139.25, 135.25, 128.85, 128.06, 127.64, 127.59, 126.43, 125.19, 123.69, 121.43, 119.37, 117.40, 114.10, 107.60, 104.79, 56.66, 56.49, 52.00.

### Synthesis of 6-benzyl-9-fluoro-2,2,8-trimethoxy-6H-dibenzo[c,e][1.2]thiazine 5,5-dioxide (SET-77C)

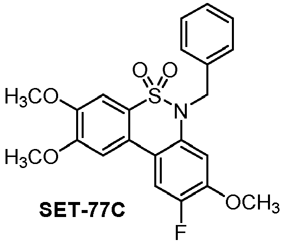

SET-77A (150 mg, 0.31 mmol), PIDA (109 mg, 0.34 mmol), I₂ (86 mg, 0.34 mmol) and K₂CO₃ (63 mg, 0.46 mmol) were used according to the general procedure C to afford compound SET-77C as a white solid (52%). ¹H NMR (500 MHz, Chloroform-d) δ 7.47 (d, J = 12.0 Hz, 1H, Ar), 7.37 (s, 1H, Ar), 7.20 (p, J = 2.3, 2.8 Hz, 3H, Ar), 7.16 (dd, J = 2.6, 7.2 Hz, 2H, Ar), 7.02 (s, 1H, Ar), 6.60 (d, J = 7.8 Hz, 1H, Ar), 4.83 (s, 2H, CH₂), 3.98 (d, J = 2.1 Hz, 6H, 2OCH₃), 3.66 (s, 3H, OCH₃). ¹³C NMR (126 MHz, CDCl₃) δ 152.85, 151.66, 149.54, 148.50, 135.91, 132.53, 128.98, 128.57, 128.35, 126.97, 125.88, 119.28, 119.23, 112.49, 108.95, 108.93, 107.41, 105.57, 56.88, 56.74, 56.53, 54-48.

### Synthesis of 6-benzyl-2,3,8-trimethoxy-9-(trifluoromethyl)-6H-dibenzo[c,e][1,2]thiazine 5,5-dioxide (SET-78C)

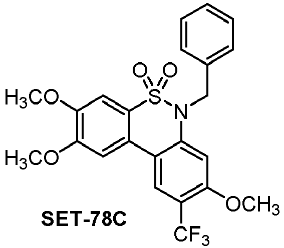

SET-78A (150 mg, 0.31 mmol), PIDA (100 mg, 0.34 mmol), I₂ (86 mg, 0.34 mmol) and K₂CO₃ (63 mg, 0.46 mmol) were used according to the general procedure C to afford compound SET-78C as a white solid (62%). ¹H NMR (500 MHz, Chloroform-d) δ 7.96 (s, 1H, Ar), 7.41 (s, 1H, Ar), 7.29 - 7.22 (m, 5H, Ar), 7.15 (s, 1H, Ar), 6.66 (s, 1H, Ar), 5.07 (s, 2H, CH₂), 4.01 (d, J = 13.9 Hz, 6H, 2OCH₃), 3.66 (s, 3H, OCH₃). ¹³C NMR (126 MHz, CDCl₃) δ 158.09, 153.03, 149.57, 136.02, 129.30, 128.41, 127.77 (2CH), 126.67, 125.68, 124.37, 124.33, 122.51, 117.05, 116.10, 115.85, 107.30, 104.97, 60.79, 56.88, 56.84, 56.33, 51.82.

### General procedure for the deprotection of methoxy groups (I)

To a solution of appropriate protected compound (1 eq.) cooled to 0°C in DCM (5-10 mL) was added BBr₃ (1M in DCM, 5 eq. per methoxy group). Reaction was allowed to warm up to room temperature and stirred (3-12h). LC/MS analysis revealed complete consumption of the starting material and formation of Demethylated N-benzyl sultam as a major product alongside with a small amount of corresponding debenzylated sultam. Reaction is cooled to 0°C and quenched with water and mixture extracted with diethyl ether (3 × 50 mL). Combined org. phases were dried over Na₂SO₄, evaporated and residue was purified via reverse phase column to afford compound.

### Synthesis of 6-benzyl-2,3,9-trihydroxy-6H-dibenzo[c,e][1,2]thiazine 5,5-dioxide (SET-69D)

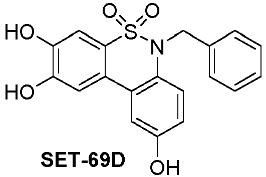

SET-69C (50 mg, 0.12 mmol) and BBr₃ (0.51 mL, 5.4 mmol) were used according to the general procedure I for the deprotection of methoxy groups. Compound SET-69D was obtained as light brown solid (52%). ¹H NMR (500 MHz, DMSO-*d₆*) δ 10.06 (bs, 2H), 9.68 (s, 1H), 7.62 (d, J = 2.9 Hz, 1H), 7.37 (m, 2H), 7.29 (d, J = 8.9 Hz, 1H), 7.26 (s, 1H), 7.20 (s, 1H), 7.06 (m, 1H), 6.99 (td, J = 2.1, 8.7 Hz, 2H), 6.69 (dt, J = 1.3, 7.0 Hz, 1H), 4.96 (s, 2H); ¹³C NMR (126 MHz, DMSO-*d₆*) δ 156.14, 153.29, 148.57, 148.46, 135.21, 130.06, 127.85, 127.29, 126.86, 126.68, 125.32, 124.64, 116.50, 111.30, 109.85, 108.26, 108.18, 104.23, 52.70.

### Synthesis of 6-benzyl-2,3,8-trihydroxy-6H-dibenzo[c,e][1,2]thiazine 5,5-dioxide (SET-67D)

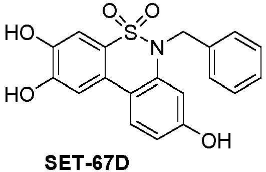

SET-67C (50 mg, 0.12 mmol) and BBr₃ (0.51 mL, 5.4 mmol) were used according to the general procedure I for the deprotection of methoxy groups. Compound SET-67D was obtained as light brown solid (61%). ¹H NMR (500 MHz, DMSO-*d₆*) δ 10.03 (s, 1H), 9.98 (s, 1H), 9.93 (s, 1H), 7.69 (d, J = 8.8 Hz, 1H), 7.24 (m, 8H), 6.70 (dd, J = 2.4, 8.6 Hz, 1H), 6.65 (d, J = 2.4 Hz, 1H), 5.03 (s, 2H) ; ¹³C NMR (126 MHz, DMSO-*d₆*) δ 158.54, 150.39, 145.57, 139.03, 137.11, 128.95, 128.92, 127.80, 127.38, 126.50, 125.20, 124.76, 116.24, 113.03, 111.62, 108.16, 107.24, 50.12.

### Synthesis of 6-benzyl-2,'3-dihydroxy-8-(trifluoromethyl)-6H-dibenzo[c,e][1,2]thiazine 5,5-dioxide (SET-62)

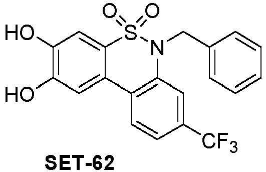

SET-61 (200 mg, 0.44 mmol) and BBr₃ (1.26 mL, 13.3 mmol) were used according to the general procedure I for the deprotection of methoxy groups. Compound SET-62 was obtained as white solid (68%). ¹H NMR (500 MHz, DMSO-*d₆*) δ 10.43 (bs, 2H, OH), 7.71 (d, J = 1.8 Hz, 1H, Ar), 7.60 (dd, J = 1.7, 8.5 Hz, 1H, Ar), 7.43 (s, 1H, Ar), 7.32 (s, 1H, Ar), 7.18 (m, 4H, Ar), 7.08 (dd, J = 2.0, 6.4 Hz, 2H, Ar), 5.20 (s, 2H, CH₂). ¹³C NMR (126 MHz, DMSO-*d₆*) δ 148.82, 145.15, 144.69, 138.59, 131.63, 130.71, 129.75, 128.61, 128.06, 127.91, 127.81, 127.51, 126.01, 124.73, 124.70, 123.84, 119.39, 116.51, 46.91.

### Synthesis of 6-benzyl-2,3,8,9-tetrahydroxy-6H-dibenzo[c,e][1,2]thiazine 5,5-dioxide (SET-09)

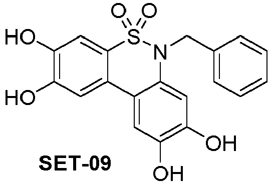

SET-08 (50 mg, 0.11 mmol) and BBr₃ (0.6 mL, 4.2 mmol) were used according to the general procedure D for the deprotection of methoxy groups. Compound SET-09 was obtained as grey solid (66%). ¹H NMR (500 MHz, DMSO-*d₆*) δ 9.98 (s, 1H, OH), 9.90 (s, 1H, OH), 9.46 (s, 1H, OH), 9.26 (s, 1H, OH), 7.21 (m, 3H, Ar), 7.16 (s, 1H, Ar), 7.12 (m, 3H, Ar), 7.06 (s, 1H, Ar), 6.63 (s, 1H, Ar), 4.82 (s, 2H, CH₂); ¹³ C NMR (101 MHz, DMSO-*d₆*) δ 149.2, 145.9, 144.8, 140.1, 134.5, 128.5, 127.3, 127.3, 125.7, 125.6, 125.6, 120.4, 114.7, 114.0, 111.4, 108.5, 52.5.

### Synthesis of 2,3,8,9-tetrahydroxy-6H-dibenzo[c,e][1,2]thiazine 5,5-dioxidee (SET-10)

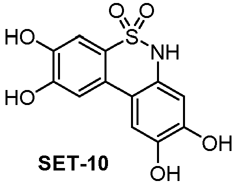

According to the general procedure E for the deprotection of benzyl groups. Compound SET-10 was obtained as beige solid (50%). ¹H NMR (500 MHz, DMSO-*d₆*) δ 1H NMR (500 MHz, DMSO-*d₆*) δ 10.34 (s, 1H, NH), 9.93 (s, 1H, OH), 9.85 (s, 1H, OH), 9.53 (s, 1H, OH), 9.00 (s, 1H, OH), 7.13 (m, 3H, Ar), 6.54 (s, 1H, Ar). ¹³C NMR (126 MHz, DMSO-*d₆*) δ 149.87, 147.06, 145.26, 142.93, 128.97, 125.37, 114.09, 110.79, 110.73, 108.02, 107.63.

### Synthesis of 2,3,7,9-tetrahydroxy-6H-dibenzo[c,e][1,2]thiazine 5,5-dioxide (SET-72)

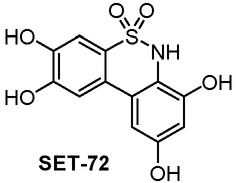

According to the general procedure E for the deprotection of methoxy and benzyl groups. Compound SET-72 was obtained as beige solid (95%). ¹H NMR (500 MHz, Methanol-*d₄*) δ 7.97 (s, NH), 7.30 (s, 1H, Ar), 7.26 (s, 1H, Ar), 6.78 (d, J = 2.5 Hz, 1H, Ar), 6.41 (d, J = 2.5 Hz, 1H, Ar); ¹³C NMR (126 MHz, MeOD) δ 155.62, 151.06, 150.36, 146.80, 128.19, 126.82, 126.32, 117.54, 112.23, 108.68, 103.48, 101.89.

### Synthesis of 6-benzyl-2,3-dihydroxy-8-(trifluoromethoxy)-6H-dibenzo[c,e][1,2]thiazine 5,5-dioxide (SET-75D)

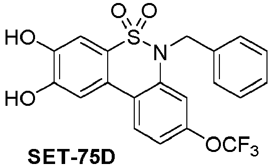

SET-75C (100 mg, 0.21 mmol) and BBr₃ (0.6 mL, 6.45 mmol) were used according to the general procedure D for the deprotection of methoxy groups. Compound SET-75D was obtained as light yellow viscous liquid (69%). ¹H NMR (500 MHz, Methanol-*d₄*) δ 7.97 (br, 2H, OH), 7.92 (d, J = 9.5 Hz, 1H, Ar), 7.33 (s, 1H, Ar), 7.28 (s, 1H, Ar), 7.20 - 7.14 (m, 5H, Ar), 7.13 - 7.08 (m, 2H, Ar), 5.05 (s, 2H, CH₂). ¹³ C NMR (126 MHz, MeOD) δ 164.68, 151.17, 149.74, 147.73, 140.01, 136.54, 129.31, 128.66, 127.77, 127.34, 126.01, 125.17, 122.62, 120.58, 118.49, 115.68, 112.88, 109.20, 52.55, 36.78.

### Synthesis of 6-benzyl-9-fluoro-2,3,8-trihydroxy-6H-dibenzo[c,e][1,2]thiazine 5,5-dioxide (SET-77D)

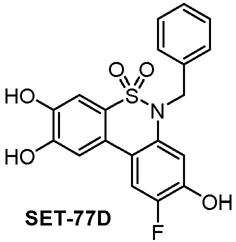

SET-77C (70 mg, 0.16 mmol) and BBr₃ (0.76 mL, 8.1 mmol) were used according to the general procedure D for the deprotection of methoxy groups. Compound SET-77D was obtained as light yellow viscous liquid (71%). ¹H NMR (500 MHz, Methanol-*d₄*) δ 7.97 (brs, 3H, OH), 7.51 (d, J = 12.2 Hz, 1H, Ar), 7.24 (s, 1H, Ar), 7.19 - 7.14 (m, 3H, Ar), 7.13 - 7.07 (m, 3H, Ar), 6.79 (d, J = 8.0 Hz, 1H, Ar), 4.89 (s, 2H, CH₂). ¹³ C NMR (126 MHz, MeOD) δ 151.90, 151.16, 149.99, 147.00, 146.88, 137.07, 135.98, 129.34, 128.91, 128.69, 127.05, 126.25, 119.73, 119.68, 112.92, 112.90, 112.69, 109.55, 54.20.

### Synthesis of 6-benzyl-2,'3-dihydroxy-8-methoxy-9-(trifluoromethyl)-6H-dibenzo[c,e][1,2]thiazine 5,5-dioxide (SET-78D)

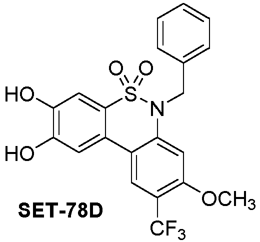

SET-78C (82 mg, 0.17 mmol) and BBr₃ (0.73 mL, 7.69 mmol) were used according to the general procedure D for the deprotection of methoxy groups. Compound SET-78D was obtained as yellow viscous liquid (68%). ¹H NMR (500 MHz, Methanol-*d₄*) δ 7.99 (br, 2H, OH), 7.98 (s, 1H, Ar), 7.32 (s, 1H, Ar), 7.29 - 7.13 (m, 6H, Ar), 6.95 (s, 1H, Ar), 5.17 (s, 2H, CH₂), 3.77 (s, 3H, OCH₃). ¹³ C NMR (126 MHz, MeOD) δ 173-01, 164.87, 158.76, 151-49, 147-38, 143.31, 137.15, 129.66, 128.89, 128.77, 127.00, 125.54, 124.53, 124.48, 118.78, 112.15, 109.27, 106.47, 61.54, 56.72, 51.77.

### General Procedure for the Synthesis of 2-bromo-4,5-dimethoxybenzenesulfonyl chloride (2)

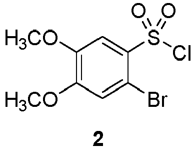

4-Bromoveratrole 1 (4.0 ml, 27.8 mmol) was added in a dropwise over 20 min. to a well stirred flask containing chlorosulfonic acid (8 ml) cooled to 0 C. The black mixture was then stirred for a further 30 min before cautious addition to ice (150 ml). After melting DCM (100ml) was added and the phase were separated. The aqueous phase was extracted with DCM (100ml) and the combination organic phases were dried over MgSO4. Filtration and solvent removal under reduced pressure gave the sulfonyl chloride 2 (7.17 g 82%) as a white solid which was further purified by recrystallisation from Diethylether. m.p. 78 C; 1H-NMR (400 MHz, CDCl₃) δ 3.94 (s, 3H), 3.99 (s, 3H), 7.18 (s, 1H), 7.59 (s, 1H) ; ¹³C-NMR (100 MHz, CDCl₃) δ 56.5, 56.7, 112.5, 113.0, 117.7, 134.6, 147.8, 154.1.

### Synthesis of 2-bromo-N-(4-fluorobenzyl)-4,5-dimethoxybenzenesulfonamide (SET-11)

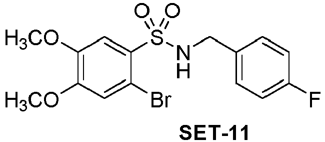

2 (10.0 g, 31.69 mmol) was added to a solution of fluorobenzylamine (4.15 mL, 38.03 mmol) and DIPEA (16.45 mL, 95.07 mmol) in DCM (30 mL) cooled to 0°C. Reaction was allowed to warm to r.t. and stirred for one hour, until complete conversion of the starting material has been confirmed by TLC (3% EtOAc in DCM). Reaction is quenched with water (30 mL). Crude product was extracted with DCM (3 × 30 mL). Combined org. Phases were washed with 1M HCl (3 × 50 mL), dried over Na₂SO₄ and evaporated to obtain the yellow oil, which was recrystallized from hot MeOH to provide compound SET-11 as a white crystalline solid (10.41 g, 85% ). ¹H NMR (400 MHz, DMSO-*d₆*) δ 8.24 (s, 1H, NH), 7.38 (s, 1H, Ar), 7.26 (s, 1H, Ar), 7.25 - 7.15 (m, 5H, Ar), 4.08 (s, 2H, CH₂), 3.84 (s, 3H, CH₃), 3.76 (s, 3H, CH₃) ; ¹³C NMR (101 MHz, DMSO-*d₆*) δ 151.7, 147.3, 137.6, 131.5, 128.0, 127.6, 127.0, 117.4, 113.3, 110.6, 56.3, 55.8, 46.1.

### General procedure for the synthesis of biaryl sulfonamides (Cross coupling)

Biaryl sulfonamides were prepared according to the slightly modified described procedure.² By using procedure B, Compound SET-11 (1.0 eq.), appropriate boronic acid (1.5 eq.), potassium carbonate (4.0 eq.) and Pd(PPh₃)₄ (0.03 eq.) were suspended in the mixture of toluene : ethanol: water/5:2:1 (13 mL). Sequential applying of N₂ flow and vacuum were used to degas the mixture before heating in a MW reactor at 120 °C for 1h. The crude product was extracted with ethyl acetate (3 × 30 mL). To the organic phase was dried over sodium sulfate. The mixture was then filtered through the bed of celite and solvent removed under reduced pressure. The residue was purified by flash chromatography using Hexane/EtOAc (gradient) to afford the biaryl sulfonamides.

### General procedure for the synthesis of sultams via cyclization of secondary sulfonamides (C)

Cyclization was achieved according to the procedure described in the literatures appropriate sulfonamide (1 eq.), PIDA (1.10 eq.), I₂ (1.10 eq.) and K₂CO₃ (1.50 eq.) were suspended in DCM (20 mL). The dark red solution was stirred at 35 °C for 1-3h, until almost complete conversion was determined by TLC (3%EtOAc in DCM). Reaction is quenched with sat. aq. Na₂S₂O₅, and mixture was stirred at the r.t. until discoloration of the solution. Crude product was extracted with DCM and purified via flash chromatography (DCM/EtOAc, gradient, 0->1% EtOAc) and triturated with MeOH to afford sultams.

### Synthesis of 6-(4-fluorobenzyl)-2,3,8-trimethoxy-6H-dibenzo[c,e][1,2]thiazine 5,5-dioxide (SET-66C)

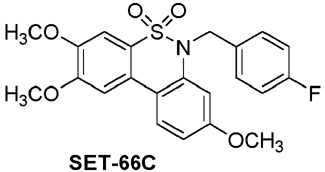

SET-66A (100 mg, 0.23 mmol), PIDA (80 mg, 0.25 mmol), I₂ (63 mg, 0.25 mmol) and K₂CO₃ (98 mg, 0.34 mmol) were used according to the general procedure C to afford compound SET-66C as a white solid (70%). ¹H NMR (500 MHz, DMSO-*d₆*) δ 8.10 (d, J = 8.8 Hz, 1H), 7.47 (s, 1H), 7.36 (s, 1H), 7.17 (m, 2H), 7.04 (m, 2H), 6.94 (d, J = 2.5 Hz, 1H), 6.91 (dd, J = 2.6, 8.7 Hz, 1H), 5.14 (s, 2H), 3.95 (s, 3H), 3.91 (s, 3H), 3.77 (s, 3H); ¹³C NMR (126 MHz, DMSO-*d₆*) δ 162.86, 160.92, 160.44, 152.77, 148.83, 138.73, 132.61, 129.97, 129.91, 127.49, 126.08, 126.02, 117.94, 115.76, 115.59, 111.80, 108.33, 106.64, 104.38, 56.64, 56.51, 56.01, 49.43.

### Synthesis of 6-(4-fluorobenzyl)-2,3,9-trimethoxy-6H-dibenzo[c,e][1,2]thiazine 5,5-dioxide (SET-68C)

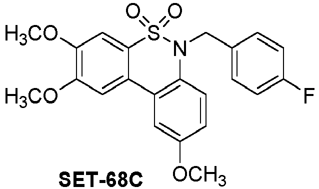

SET-68A (200 mg, 0.46 mmol), PIDA (160 mg, 0.50 mmol), I₂ (126 mg, 0.50 mmol) and K₂CO₃ (95.9 mg, 0.69 mmol) were used according to the general procedure C to afford compound SET-68C as mixture of two positional isomers a white solid, purified by flash chromatography using (DCM/EtOAc, gradient, o->i% EtOAc) to afford (55% SET-69C) and 35% other Isomer. ¹H NMR (500 MHz, DMSO-*d₆*) δ 7.61 (d, J = 2.8 Hz, 1H, Ar), 7.58 (dd, J = 1.2, 8.2 Hz, 1H, Ar), 7.48 (s, 1H, Ar), 7.39 (t, J = 8.1 Hz, 1H, Ar), 7.34 (s, 1H, Ar), 7.33 (d, J = 9.0 Hz, 1H, Ar), 7.23 (m, 1H, Ar), 7.19 (dd, J = 1.1, 8.4 Hz, 1H, Ar), 6.98 (m, 1H, Ar), 4.93 (s, 2H), 3.95 (d, J = 2.3 Hz, 6H), 3.92 (s, 3H); ¹³C NMR (126 MHz, DMSO-*d₆*) δ 161.81, 156.33, 153.30, 148.60, 131.09, 129.93, 127.21, 126.68, 125.28, 124.67, 116.51, 114.62, 113.55, 111.37, 109.85, 108.31, 104.35, 55.68, 55.57, 55.03, 50.83.

### Synthesis of 6-(4-fluorobenzyl)-2,3,7-trimethoxy-6H-dibenzo[c,e][1,2]thiazine 5,5-dioxide (Isomer of SET-68C)

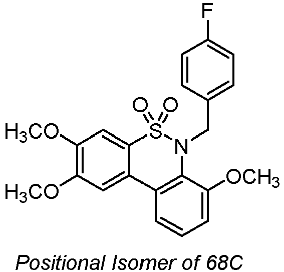

Yield: 35%. ¹H NMR (500 MHz, DMSO-*d₆*) δ 7.61 (d, J = 2.8 Hz, 1H), 7.58 (dd, J = 1.2, 8.2 Hz, 1H), 7.48 (s, 1H), 7.39 (t, J = 8.1 Hz, 1H), 7.34 (s, 1H), 7.33 (d, J = 9.0 Hz, 1H), 7.23 (d, J = 15.9 Hz, 2H), 7.19 (dd, J = 1.1, 8.4 Hz, 1H), 7.04 (m, 3H), 6.98 (m, 2H), 6.73 (m, 4H), 4.93 (s, 2H), 4.73 (s, 2H), 3.95 (d, J = 2.3 Hz, 6H), 3.92 (s, 3H), 3.90 (s, 3H), 3.85 (d, J = 2.9 Hz, 6H); ¹³C NMR (126 MHz, DMSO-*d₆*) δ 7.58 (dd, J = 1.2, 8.2 Hz, 1H, Ar), 7.48 (s, 1H), 7.39 (t, J = 8.1 Hz, 1H), 7.23 (m, 1H, Ar), 7.19 (dd, J = 1.1, 8.4 Hz, 1H, Ar), 7.04 (m, 1H), 6.98 (m, 1H), 6.73 (m, 2H), 4.93 (s, 2H), 4.73 (s, 2H), 3.90 (s, 3H), 3.85 (d, J = 2.9 Hz, 6H);

### Synthesis of 6-(4-fluorobenzyl)-2,3-dimethoxy-8-(trifluoromethyl)-6H-dibenzo[c,e][1,2]thiazine 5,5-dioxide (SET-58)

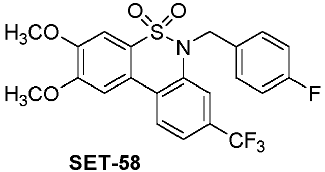

SET-25 (200 mg, 0.46 mmol), PIDA (160 mg, 0.50 mmol), I₂ (126 mg, 0.50 mmol) and K₂CO₃ (95.9 mg, 0.69 mmol) were used according to the general procedure C to afford compound SET-58 as a white solid (66%). ¹H NMR (500 MHz, DMSO-*d₆*) δ 8.43 (d, J = 8.3 Hz, 1H), 7.81 (d, J = 1.7 Hz, 1H), 7.67 (dd, J = 1.7, 8.4 Hz, 1H), 7.63 (s, 1H), 7.45 (s, 1H), 7.11 (m, 2H), 7.03 (m, 2H), 5.24 (s, 2H), 3.97 (d, J = 4.4 Hz, 6H); ¹³C NMR (126 MHz, DMSO-*d₆*) δ 162.94, 161.00, 152.78, 150.50, 137.69, 131.99, 130.06, 130.00, 128.79, 128.12, 127.42, 124.30, 121.83, 119.12, 115.84, 115.67, 109.60, 104.56, 56.82, 56.71, 49.91.

### Synthesis of 6-(4-fluorobenzyl)-2,3,8,9-tetramethoxy-6H-dibenzo[c,e][1,2]thiazine 5,5-dioxide (SET-16)

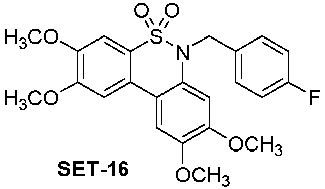

SET-12 (200 mg, 0.43 mmol), PIDA (150 mg, 0.47 mmol), I₂ (110 mg, 0.47 mmol) and K₂CO₃ (80 mg, 0.64 mmol) were used according to the general procedure C to afford compound SET-16 as a white solid (55%). ¹H NMR (500 MHz, Chloroform-*d*) δ 7.37 (s, 1H), 7.16 (s, 1H), 7.08 (dd, J = 5.4, 8.6 Hz, 2H), 7.03 (s, 1H), 6.85 (t, J = 8.6 Hz, 2H), 6.58 (s, 1H), 4.76 (s, 2H), 4.00 (d, J = 5.8 Hz, 6H), 3.95 (s, 3H), 3.74 (s, 3H); ¹³C NMR (126 MHz, CDCl₃) δ 163.34, 161.38, 152.38, 150.03, 148.91, 147.71, 131.91, 131.16, 131.14, 130.21, 130.14, 126.75, 126.39, 119.23, 115.30, 115.13, 107-53, 107-37, 107.12, 105.41, 56.48, 56.40, 55.97, 54.14.

### Synthesis of 6-(4-fluorobenzyl)-2,3,7,9-tetramethoxy-6H-dibenzo[c,e][1,2]thiazine 5,5-dioxide (SET-70C)

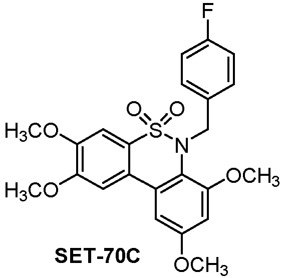

SET-70A (170 mg, 0.36 mmol), PIDA (128 mg, 0.40 mmol), I₂ (101 mg, 0.40 mmol) and K₂CO₃ (74 mg, 0.54 mmol) were used according to the general procedure C to afford compound SET-70C as a white solid (68%). ¹H NMR (500 MHz, DMSO-*d₆*) δ 7.21 (s, 1H), 7.18 (s, 1H), 7.03 (d, J = 2.5 Hz, 1H), 6.75 (d, J = 2.5 Hz, 1H), 6.72 (dd, J = 2.9, 7.4 Hz, 4H), 4.64 (s, 2H), 3.94 (s, 3H), 3.89 (s, 3H), 3.88 (s, 3H), 3.86 (s, 3H); ¹³C NMR (126 MHz, DMSO-*d6*) δ 161.77, 159.83, 158.22, 154.52, 151.21, 148.60, 130.14, 130.07, 129.29, 129.05, 127.44, 125.38, 118.37, 113.49, 113.32, 108.56, 104.49, 100.69, 98.98, 55.66, 55.48, 55.10, 52.27.

### Synthesis of 6-(4-fluorobenzyl)-2,3,8-trihydroxy-6H-dibenzo[c,e][1,2]thiazine 5,5-dioxidedioxide (SET-66D)

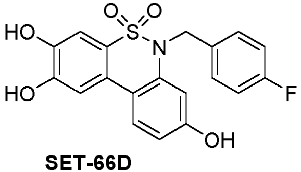

SET-66C (55 mg, 0.13 mmol) and BBr₃ (0.45mL, 5.4 mmol) were used according to the general procedure I for the deprotection of methoxy groups. Compound SET-66D was obtained as a grey solid (61%). ¹H NMR (500 MHz, DMSO-*d₆*) δ 9.89 (s, 2H, OH), 9.69 (s, 1H, OH), 7.68 (d, J = 8.7 Hz, 1H, Ar), 7.24 (s, 1H, Ar), 7.19 (m, 3H, Ar), 7.09 (m, 2H, Ar), 6.71 (dd, J = 2.4, 8.6 Hz, 1H, Ar), 6.65 (d, J = 2.4 Hz, 1H, Ar), 4.99 (s, 2H, CH₂) ; ¹³C NMR (126 MHz, DMSO-*d₆*) δ 160.78, 148.86, 145.15, 144.65, 134.82, 131.64, 130.69, 129.93, 129.86, 129.65, 128.08, 127.83, 126.00, 124.77, 124.74, 124.71, 124.68, 123.84, 119.39, 116.52, 115.40, 115.23, 45.52.

### Synthesis of 6-(4-fluorobenzyl)-2,3,9-trihydroxy-6H-dibenzo[c,e][1,2]thiazine 5,5-dioxide (SET-68D)

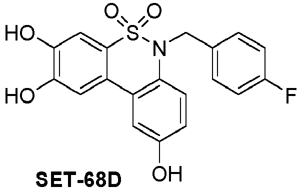

SET-68C (40 mg, 0.09 mmol) and BBr₃ (0.38 mL, 4.1 mmol) were used according to the general procedure I for the deprotection of methoxy groups. Compound SET-68D was obtained as a light Brown solid (55%). ¹H NMR (500 MHz, DMSO-*d₆*) δ 10.05 (s, 2H, OH), 9.69 (s, 1H, OH), 7.19 (s, 1H, Ar), 7.14 (d, J = 8.8 Hz, 1H, Ar), 7.12 (s, 1H, Ar), 7.09 (d, J = 2.7 Hz, 1H, Ar), 7.03 (dd, J = 2.7, 5.8 Hz, 2H, Ar), 6.98 (m, 2H, Ar), 6.76 (dd, J = 2.7, 8.7 Hz, 1H, Ar), 4.80 (s, 2H, CH₂).

### Synthesis of 6-(4-fluorobenzyl)-2,2-dihydroxy-8-(trifluoromethyl)-6H-dibenzo[c,e][1,2]thiazine 5,5-dioxide (SET-59)

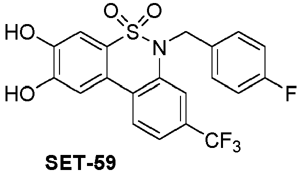

SET-58 (30 mg, 0.06 mmol) and BBr₃ (0.12 mL, 0.64 mmol) were used according to the general procedure I for the deprotection of methoxy groups. Compound SET-59 was obtained as light brown solid (68%). ¹H NMR (500 MHz, DMSO-*d₆*) δ 10.38 (s, 2H, OH), 8.10 (d, J = 8.4 Hz, 1H, Ar), 7.74 (d, J = 1.7 Hz, 1H, Ar), 7.61 (dd, J = 1.8, 8.5 Hz, 1H, Ar), 7.41 (s, 1H, Ar), 7.30 (s, 1H, Ar), 7.10 (dd, J = 5.6, 8.7 Hz, 2H, Ar), 7.02 (m, 2H, Ar), 5.18 (s, 2H, CH₂) ; ¹³C NMR (126 MHz, DMSO-*d₆*) δ 160.78, 148.86, 145-15, 144.65, 134.82, 131.64, 130.69, 129.93, 129.86, 129.65, 128.08, 127.83, 126.00, 124.77, 124.74, 124.71, 124.68, 123.84, 119.39, 116.52, 115.40, 115.23, 45.52.

### Synthesis of 6-(4-fluorobenzyl)-2,3,8,9-tetrahydroxy-6H-dibenzo[c,e][1,2]thiazine 5,5-dioxide (SET-17)

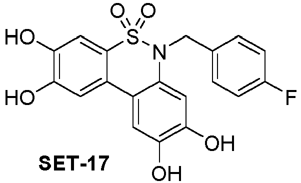

SET-16 (260 mg, 0.56 mmol) and BBr₃ (2.13 mL, 22.1 mmol) were used according to the general procedure I for the deprotection of methoxy groups. Compound SET-17 was obtained as light yellow solid (62%). ¹H NMR (400 MHz, DMSO-*d₆*) δ 9.99 (s, 1H, OH), 9.91 (s, 1H, OH), 9.49 (s, 1H, OH), 9.30 (s, 1H, OH), 7.14 (s, 1H, Ar), 7.09 (d, J = 2.0 Hz, 2H, Ar), 7.03 (m, 4H, Ar), 6.63 (s, 1H, Ar), 4.77 (s, 2H, CH₂).

### Synthesis of 2,3,7,9-tetrahydroxy-6H-dibenzo[c,e][1,2]thiazine 5,5-dioxide (SET-72)

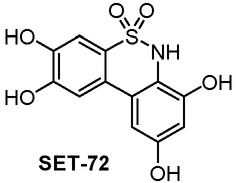

According to the general procedure E for the deprotection of methoxy and benzyl groups. Compound SET-72 was obtained as beige solid (95%). ¹H NMR (500 MHz, Methanol-*d₄*) δ 7.97 (s, NH), 7.30 (s, 1H, Ar), 7.26 (s, 1H, Ar), 6.78 (d, J = 2.5 Hz, 1H, Ar), 6.41 (d, J = 2.5 Hz, 1H, Ar); ¹³C NMR (126 MHz, MeOD) δ 155.62, 151.06, 150.36, 146.80, 128.19, 126.82, 126.32, 117.54, 112.23, 108.68, 103.48, 101.89.

### Synthesis of biaryl sulfonamides (Cross coupling)

Biaryl sulfonamides were prepared according to the slightly modified described procedure.² By using procedure A to obtain SET-05 then procedure B, Compound SET-05 (1.0 eq.), appropriate boronic acid (1.5 eq.), potassium carbonate (4.0 eq.) and Pd(PPh₃)₄ (0.03 eq.) were suspended in the mixture of toluene : ethanol : water/5:2:1 (13 mL). Sequential applying of N₂ flow and vacuum were used to degas the mixture before heating in a MW reactor at 120 °C for 1h. The crude product was extracted with ethyl acetate (3 × 30 mL). To the organic phase was dried over sodium sulfate. The mixture was then filtered through the bed of celite and solvent removed under reduced pressure. The residue was purified by flash chromatography using Hexane/EtOAc (gradient) to afford the biaryl sulfonamides.

### Synthesis of 3',4,4',5-tetramethoxy-N-(4-methoxybenzyl)-[1,1'-biphenyl]-2-sulfonamide (SET-14)

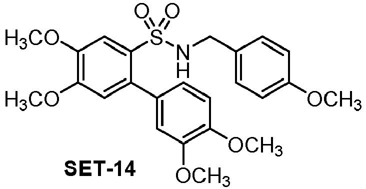

SET-05 (400 mg, 0.98 mmol), 3,4-dimethoxybenzeneboronic acid (224 mg, 1.48 mmol), K₂CO₃ (540 mg, 3.92 mmol) and Pd(PPh₃)₄ (23 mg, 0.02 mmol) were used according to the general procedure B to afford compound SET-14 as white solid (77%). ¹H NMR (500 MHz, Chloroform-*d*) δ 7.66 (s, 1H, NH), 7.08 (d, J = 2.1 Hz, 1H, Ar), 6.96 (m, 2H, Ar), 6.91 (dd, J = 2.1, 8.2 Hz, 1H, Ar), 6.83 (d, J = 8.2 Hz, 1H, Ar), 6.79 (s, 1H, Ar), 6.76 (m, 3H, Ar), 4.00 (s, 3H, CH₃), 3.94 (s, 3H, CH₃), 3.87 (s, 3H, CH₃), 3.83 (s, 3H, CH₃), 3.76 (s, 3H, CH₃), 3.73 (d, J = 3.6 Hz, 2H, CH₂); ¹³C NMR (126 MHz, CDCl₃) δ 159.28, 151.46, 149.09, 148.28, 147.83, 133.53, 130.89, 129.79, 129.24, 127.99, 121.45, 114.61, 113.92, 113.11, 112.26, 110.74, 56.41, 56.30, 55.98, 55.95, 55.31, 46.90.

### Synthesis of sultams via cyclization of secondary sulfonamides (C)

Cyclization was achieved according to the procedure described in the literatures appropriate sulfonamide (1 eq.), PIDA (1.10 eq.), I₂ (1.10 eq.) and K₂CO₃ (1.50 eq.) were suspended in DCM (20 mL). The dark red solution was stirred at 35 °C for 1-3h, until almost complete conversion was determined by TLC (3%EtOAc in DCM). Reaction is quenched with sat. aq. Na₂S₂O₅, and mixture was stirred at the r.t. until discoloration of the solution. Crude product was extracted with DCM and purified via flash chromatography (DCM/EtOAc, gradient, 0->1% EtOAc) and triturated with MeOH to afford sultams.

### Synthesis of 2,3,8,9-tetramethoxy-6-(4-methoxybenzyl)-6H-dibenzo[c,e][1,2] thiazine 5,5-dioxide (SET-18)

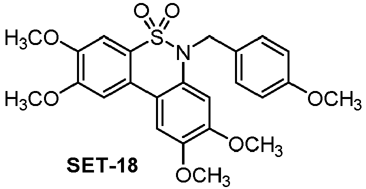

SET-14 (100 mg, 0.23 mmol), PIDA (80 mg, 0.25 mmol), I₂ (63 mg, 0.25 mmol) and K₂CO₃ (98 mg, 0.34 mmol) were used according to the general procedure C to afford compound SET-18 as a white solid (70%). ¹H NMR (500 MHz, DMSO-*d₆*) δ 7.48 (s, 1H, Ar), 7.39 (s, 1H, Ar), 7.31 (s, 1H, Ar), 6.97 (s, 1H, Ar), 6.93 (m, 2H, Ar), 6.67 (m, 2H, Ar), 4.92 (s, 2H, CH₂), 3.94 (s, 3H, CH₃), 3.91 (s, 3H, CH₃), 3.88 (s, 3H, CH₃), 3.75 (s, 3H, CH₃), 3.62 (s, 3H, CH₃); ¹³C NMR (126 MHz, DMSO-*d₆*) δ 159.08, 152.52, 150.11, 148.86, 147.17, 131.35, 129.88 (2CH), 127.74 (2CH), 126.68, 126.31 (2CH), 118.70, 113.94, 108.81, 107.15, 104.79, 56.73, 56.65, 56.49, 56.26, 55-44, 51.85.

### General Procedure for the Synthesis of 2-bromo-4,5-dimethoxybenzenesulfonyl chloride (2)

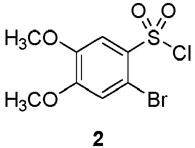

4-Bromoveratrole 1 (4.0 ml, 27.8 mmol) was added in a dropwise over 20 min. to a well stirred flask containing chlorosulfonic acid (8 ml) cooled to 0 C. The black mixture was then stirred for a further 30 min before cautious addition to ice (150 ml). After melting DCM (100ml) was added and the phase were separated. The aqueous phase was extracted with DCM (100ml) and the combination organic phases were dried over MgSO₄. Filtration and solvent removal under reduced pressure gave the sulfonyl chloride 2 (7.17 g 82%) as a white solid which was further purified by recrystallisation from Diethylether. m.p. 78 C; ¹H-NMR (400 MHz, CDCl₃) δ 3.94 (s, 3H), 3.99 (s, 3H), 7.18 (s, 1H), 7.59 (s, 1H) ; ¹³C-NMR (100 MHz, CDCl₃) δ 56.5, 56.7, 112.5, 113.0, 117.7, 134.6, 147.8, 154.1.

### General Procedure for the Synthesis of 2-bromo-4,5-dimethoxy-N-(prop-2-yn-1-yl)benzene sulfonamide (SET-23)

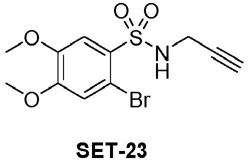

2-bromo-4,5-dimethoxybenzene-1-sulfonyl chloride 2 (1.0 eq.) was added to the stirred solution of corresponding propargyl amine (1.5 eq.) and DIPEA in dry DCM (20 mL) cooled to 0°C. Reaction was allowed to warm to r.t. and stirred until complete consumption of the starting material was determined by TLC (Hexane : EtOAc / 3:2), 6h. Reaction is quenched with water (20 mL) and crude product extracted with DCM (3×20 mL). Combined organic phases were washed with 1M HCl (3×60 mL) and brine (3×60 mL), and then dried over anhydrous Na₂SO₄. Solvent was removed under reduced pressure to afford crude sulfonamide, which was purified by column chromatography using silica and hexanes/EtOAc 3:1 → 2:1 to obtain desired product (85%). ¹H NMR (500 MHz, DMSO-*d₆*) δ 8.16 (s, 1H, NH), 7.49 (s, 1H, Ar), 7.32 (s, 1H, Ar), 3.86 (s, 3H, CH₃), 3.82 (s, 3H, CH₃), 3.77 (d, J = 2.6 Hz, 2H, CH₂), 3.06 (t, J = 2.5 Hz, 1H, CH) ; ¹³C NMR (126 MHz, DMSO-*d₆*) δ 152.36, 147.80, 131.72, 117.93, 113.80, 111.37, 79.90, 74.89, 56.82, 56.38, 32.26.

### General Procedure for the Synthesis of azides of heterocycles, alkyl and benzyl compounds

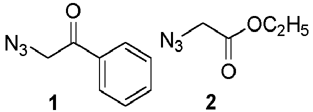

(Method A): A round bottom flask was charged with alpha-halogenated heterocycles (6.5 mmol) in acetonitrile (20 mL) at room temperature. Sodium azide (19.4 mmol) was added to the stirring solution for 4 h. Upon completion, cold water was added and the reaction mixture was extracted with EtOAc (2×20 mL). The organic layers were then dried using anhydrous MgSO₄, filtered, and concentrated to obtain corresponding azides with good yield.

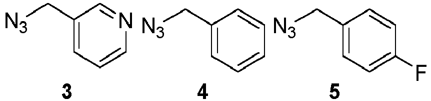

(Method b): To a stirred solution of 10.0 mmol benzyl halides in 100 mL of acetone/H₂O 4:1 (*v*/*v*) was added 15.0 mmol (0.98 g) of sodium azide. The reaction mixture was stirred at room temperature for 24 h. After, the reaction was extracted with Et₂O (3 × 50 mL), dried over Na₂SO₄, and concentrated under reduced pressure to give benzyl azides as pale-yellow oils.

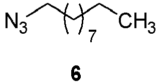

(Method C): To a stirred solution of 10.0 mmol alkyl halides in 20 mL of DMF, 12.0 mmol (0.78 g) of sodium azide was added. The reaction mixture was stirred at 70 °C for 24 h in an oil bath. After, the reaction was extracted with Et₂O (3 × 50 mL), dried over Na₂SO₄, and concentrated under reduced pressure to give alkyl azides as pale yellow oil.

### General Procedure for the Synthesis of triazole analogues of 2-bromo-4.5-dimethoxy-N-(prop-2-yn-1-yl)benzene sulfonamide

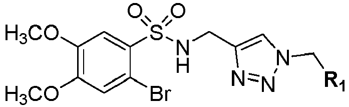

To a solution of SET-23 (0.2 g, 0.97 mmol) in mixture of DMF: water (3: 2, v/v, 5 mL), a stoichiometric amount of corresponding acetophenone and heterocyclic azides (1.0 mmol) was added. After addition of copper sulphate (0.48 mmol) and sodium l-ascorbate (0.73 mmol), reaction mixture was left for stirring at room temperature for 12 h. On complete utilization of starting material, reaction mixture was adsorbed directly on silica, and subjected to purification by column chromatography to obtain desired products in 65-94% yields (Eluent: hexane: ethyl acetate: 8.0: 2.0 - 1.0: 9.0).

### Synthesis of 2-bromo-4,5-dimethoxy-N-((1-(2-xox-2-phenylethyl)-1H-1,2,3-triazol-4-yl)methyl) benzenesulfonamide (SET-27A)

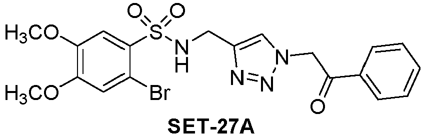

Following General Procedure, 2-bromo-4,5-dimethoxy-*N*-(prop-2-yn-1-yl)benzene sulfonamide (SET-23) reacted with 2-azido-1-phenylethan-1-one to afford (70 %) of the title compound as a pale yellow solid after column chromatography (EtOAc in Hexane : 0-70%). ¹H NMR (500 MHz, DMSO-*d₆*) δ 8.27 (t, J = 6.1 Hz, 1H, NH), 8.06 (m, 2H, Ar), 7.86 (s, 1H, Ar), 7.74 (t, J = 7.4 Hz, 1H, Ar), 7.61 (m, 2H, Ar), 7.47 (s, 1H, Ar), 7.31 (s, 1H, Ar), 6.12 (s, 2H, CH₂), 4.20 (d, J = 6.0 Hz, 2H, CH₂), 3.86 (s, 3H, CH₃), 3.81 (s, 3H, CH₃); ¹³C NMR (126 MHz, DMSO-*d₆*) δ 192.54, 152.23, 147.88, 144.13, 134.67, 134.62, 131.67, 129.45, 128.63, 125.41, 118.03, 113.63, 111.00, 56.78, 56.33, 56.17, 38.47.

### Synthesis ofN-((1-benzyl-1H-1,2,3-triazol-4-yl)methyl)-2-bromo-4,5-dimethoxybenzenesulfonamide (SET-35A)

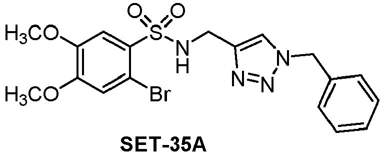

Following General Procedure, 2-bromo-4,5-dimethoxy-*N*-(prop-2-yn-1-yl)benzene sulfonamide (SET-23) reacted with Benzyl azide to afford (78 %) of the title compound as a white solid after column chromatography (EtOAc in Hexane : 0-60%). ¹H NMR (500 MHz, DMSO-*d₆*) δ 8.19 (d, J = 5.3 Hz, 1H, NH), 7.86 (s, 1H, Ar), 7.42 (s, 1H, Ar), 7.35 (m, 3H, Ar), 7.25 (m, 3H, Ar), 5.51 (s, 2H, CH₂), 4.14 (d, J = 4.5 Hz, 2H, CH₂), 3.83 (s, 3H, CH₃), 3.78 (s, 3H, CH₃); ¹³C NMR (126 MHz, DMSO-*d₆*) δ 152.19, 147.83, 144.27, 136.41, 131.66, 129.19, 128.58, 128.35, 123.86, 117.89, 113.64, 110.95, 56.79, 56.30, 53.10, 38.35.

### Synthesis of 2-bromo-N-((1-(4-fluorobenzyl)-1H-1,2,3-triazol-4-yl)methyl)-4,5-dimethoxy benzenesulfonamide (SET-80A)

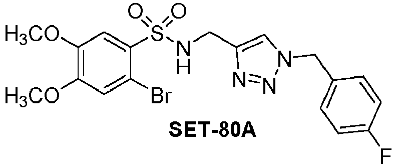

Following General Procedure, 2-bromo-4,5-dimethoxy-*N*-(prop-2-yn-1-yl)benzene sulfonamide (SET-23) reacted with fluorobenzyl azide to afford (75 %) of the title compound as a white solid after column chromatography (EtOAc in Hexane : 0-75%). ¹H NMR (500 MHz, DMSO-*d₆*) δ 8.19 (t, J = 5.9 Hz, 1H, NH), 7.87 (s, 1H, Ar), 7.41 (s, 1H, Ar), 7.32 (m, 2H, Ar), 7.25 (s, 1H, Ar), 7.21 (m, 2H, Ar), 5.51 (s, 2H, CH₂), 4.14 (d, J = 5.7 Hz, 2H, CH₂), 3.84 (s, 3H, CH₃), 3.78 (s, 3H, CH₃); ¹³C NMR (126 MHz, DMSO-*d₆*) δ 172.51, 162.79, 161.36, 152.18, 147.82, 144.32, 132.69, 132.67, 131.68, 130.71, 130.64, 123.78, 117.88, 116.10, 115.93, 113.62, 110.94, 56.78, 56.28, 52.29, 38.34.

### Synthesis of 2-bromo-4,5-dimethoxy-N-((1-(pyridin-3-ylmethyl)-1H-1,2,3-triazol-4-yl)methyl)benzene sulfonamide (SET-51A)

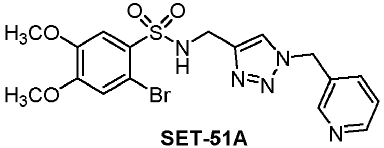

Following General Procedure, 2-bromo-4,5-dimethoxy-*N*-(prop-2-yn-1-yl) benzene sulfonamide (SET-23) reacted with 3-(azidomethyl) pyridine to afford (80 %) of the title compound as a white solid after column chromatography (EtOAc in Hexane : 0-90%). ¹H NMR (500 MHz, Chloroform-d) δ 8.61 (dd, J = 4.9, 1.6 Hz, 1H), 8.56 (d, J = 2.3 Hz, 1H), 7.56 (d, J = 3.8 Hz, 2H), 7.43 (s, 1H), 7.36 - 7.29 (m, 1H), 7.07 (s, 1H), 5.90 (t, J = 6.3 Hz, 1H), 5.49 (s, 2H), 4.21 (d, J = 6.2 Hz, 2H), 3.91 (d, J = 9.6 Hz, 6H); ¹³C NMR (126 MHz, CDCl₃) δ 152.50, 150.23, 149.03, 148.02, 144.40, 135.85, 130.29, 130.08, 124.05, 122.27, 117.11, 113.81, 111.32, 56.62, 56.49, 51.57, 38.74.

### Synthesis of 2-bromo-N-((1-decyl-1H-1,2,3-triazol-4-yl)methyl)-4,5-dimethoxybenzenesulfonamide (SET-43A)

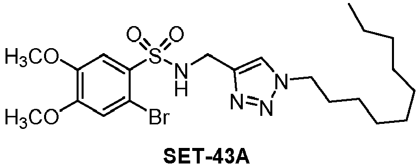

Following general procedure G, 2-bromo-4,5-dimethoxy-*N*-(prop-2-yn-1-yl)benzene sulfonamide (SET-23) reacted with 1-azidodecane to afford (88 %) of the title compound as a white solid after column chromatography (EtOAc in Hexane : 0-65%). ¹H NMR (500 MHz, DMSO-*d₆*) δ 8.17 (t, 1H, NH), 7.75 (s, 1H, Ar), 7.40 (s, 1H, Ar), 7.25 (s, 1H, Ar), 4.22 (t, J = 7.2 Hz, 2H, CH₂), 4.14 (d, J = 5.1 Hz, 2H, CH₂), 3.84 (s, 3H, -OCH3), 3.79 (s, 3H, -OCH3), 1.68 (p, J = 7.3 Hz, 2H, CH₂), 1.24 (m, 14H, CH₂), 0.85 (t, J = 6.8 Hz, 3H, CH₃); ¹³C NMR (126 MHz, DMSO-*d₆*) δ 152.14, 147.78, 143.64, 131.82, 123.46, 117.80, 113.62, 110.95, 56.77, 56.28, 49.58, 38.37, 31.77, 30.17, 29.40, 29.34, 29.15, 28.83, 26.25, 22.58, 14.43.

### General procedure for the synthesis of biaryl sulfonamides (Cross coupling)

Triazole containing Biaryl sulfonamides were prepared according to the slightly modified described procedure.¹ Compound SET-43A, 80A (1.0 eq.), appropriate boronic acid (3.0 eq.), potassium carbonate (6.0 eq.) and Pd(PPh₃)₄ (0.04 eq.) were suspended in the mixture of toluene:ethanol:water/5:2:1 (13 mL). Sequential applying of N₂ flow and vacuum were used to degas the mixture and heated at 120 °C for 24h and reaction was monitored via LCMS/MS. The crude product was extracted with ethyl acetate (3 × 30 mL). To the organic phase was dried over sodium sulfate. The mixture was then filtered through the bed of celite and solvent removed under reduced pressure. The residue was purified by flash chromatography using Hexane/EtOAc (gradient) to afford the desired products.

### Synthesis of N-((1-decyl-1H-1,2,3-triazol-4-yk)methyl)-3',4,4',5-tetramethoxy-[1,1'-biphenyl]-2-sulfonamide (SET-12A)

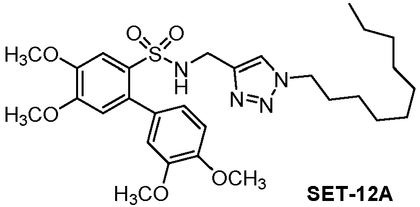

SET-43A (100 mg, 0.238 mmol), 3,4-Dimethoxybenzeneboronic acid (135 mg, 0.715 mmol), K₂CO₃ (164 mg, 1.19 mmol) and Pd(PPh₃)₄ (14 mg, 0.012 mmol) were used according to the general procedure to afford compound SET-12A as a white solid (12%). ¹H NMR (500 MHz, DMSO-*d₆*) δ 7.73 (s, 1H, Ar), 7.47 (t, J = 6.0 Hz, 1H, NH), 7.42 (s, 1H, Ar), 6.98 (d, J = 2.0 Hz, 1H, Ar), 6.94 (d, J = 8.3 Hz, 1H, Ar), 6.89 (dd, J = 2.0, 8.2 Hz, 1H, Ar), 6.80 (s, 1H, Ar), 4.24 (t, J = 7.2 Hz, 2H, CH₂), 3.94 (d, J = 5.9 Hz, 2H, CH₂), 3.82 (d, J = 3.4 Hz, 6H, CH₃), 3.78 (s, 3H, CH₃), 3.73 (s, 3H, CH₃), 1.69 (m, 2H, CH₂), 1.23 (m, 14H, CH₂), 0.86 (t, J = 2.1 Hz, 3H, CH₃); ¹³C NMR (126 MHz, DMSO-*d₆*) δ 172.50, 164.24, 148.62, 148.04, 143.87, 130.90, 123.47, 122.15, 115.77, 114.14, 111.92, 111.43, 70.47, 70.25, 60.68, 56.31, 55.95, 55.81, 49.63, 38.32, 31.75, 30.17, 29.39, 29.33, 29.14, 28.83, 26.26, 22.57, 21.53, 19.30, 14.43.

### Synthesis of N-((1-decyl-1H-1,2,3-triazol-4-yl)methyl)-4,5-dimethoxy-4'-(trifluoromethyl)-[1,1'-biphenyl]-2-sulfonamide (SET-43)

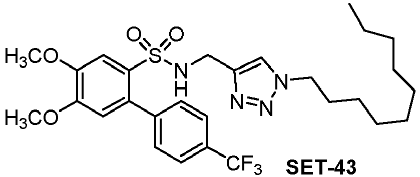

SET-43A (100 mg, 0.238 mmol), 4-Trifluoromethylbenzeneboronic acid (135 mg, 0.715 mmol), K₂CO₃ (164 mg, 1.19 mmol) and Pd(PPh₃)₄ (14 mg, 0.012 mmol) were used according to the general procedure to afford compound SET-43 as a white solid (17%). ¹H NMR (500 MHz, DMSO-*d₆*) δ 7.90 (t, J = 6.0 Hz, 1H, NH), 7.75 (s, 1H, Ar), 7.72 (d, J = 8.1 Hz, 2H, Ar), 7.57 (d, J = 8.0 Hz, 2H, Ar), 7.42 (s, 1H, Ar), 6.83 (s, 1H, Ar), 4.23 (t, J = 7.2 Hz, 2H, CH₂), 4.00 (d, J = 5.9 Hz, 2H, CH₂), 3.83 (s, 3H, CH₃), 3.81 (s, 3H, CH₃), 1.69 (p, J = 7.3 Hz, 2H, CH₂), 1.23 (d, J = 6.5 Hz, 14H, CH₂), 0.84 (t, J = 6.9 Hz, 3H, CH₃); ¹³C NMR (126 MHz, DMSO-*d₆*) δ 172.50, 148.12, 144-53, 143-74, 133.09, 130.99, 130.77, 124-74, 123-52, 115.22, 111.77, 56.42, 56.31, 49.62, 38.26, 31.75, 30.17, 29.38, 29.33, 29.14, 28.82, 26.24, 22.56, 21.53, 14.42.

### Synthesis of N-((1-(4-fluorobenzyl)-1H-1,2,3-triazol-4-yl)methyl)-3',4,5-trimethoxy-[1,1'-biphenyl]-2-sulfonamide (SET-81A)

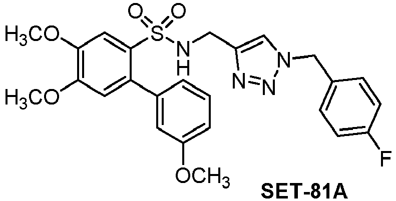

SET-80A (100 mg, 0.338 mmol), 3-Methoxybenzeneboronic acid (130 mg, 0.715 mmol), K₂CO₃ (160 mg, 1.19 mmol) and Pd(PPh₃)₄ (12 mg, 0.012 mmol) were used according to the general procedure to afford compound SET-81A as a white solid (15%). ¹H NMR (500 MHz, DMSO-*d₆*) δ 7.85 (s, 1H, Ar), 7.65 (t, J = 6.0 Hz, 1H, NH), 7.43 (s, 1H, Ar), 7.34 (m, 2H, Ar), 7.26 (t, J = 7.9 Hz, 1H, Ar), 7.20 (m, 2H, Ar), 6.92 (m, 3H, Ar), 6.81 (s, 1H, Ar), 5.52 (s, 2H, CH₂), 3.93 (d, J = 5.9 Hz, 2H, CH₂), 3.82 (d, J = 8.6 Hz, 6H, CH₃), 3.74 (s, 3H, CH₃); ¹³C NMR (126 MHz, DMSO-*d₆*) δ 171.42, 160.28, 157.68, 149.96, 146.65, 143.44, 140.35, 133.45, 131.63, 129.75, 129.68, 129.61, 127.88, 122.65, 121.24, 115.03, 114.86, 114.65, 114.43, 112.19, 110.63, 55.27, 55.22, 54.33, 51.24, 37.24.

### Results

The synthesized target compounds were evaluated in regard to their ability to inhibit PK activity, lower TAG levels and their effect on cell viability. The synthesized target compounds were divided into five batches and are presented separately below.

The effect of the target compounds was studied on liver cell line HepG2. HepG2 expresses two different pyruvate kinases (PK), pyruvate kinase muscle (PKM₂) and pyruvate kinase liver (PKL) [18]. While most cells express PKM, liver cells further express PKL. To evaluate the effect of the synthesized target compounds on the PKL expression and activity, PKM2 CRISPR Knock-out HepG2 (HepG2 KO) cells were used, hence the expression of PKM2 in these cells was knocked out, see Fig. 7A. In addition to HepG2 KO cell, HepG2 wild-type (HepG2 WT) cells were also used.

### Batch 1

HepG2 KO and HepG2 WT protein lysates and cells were treated with target compounds SET-08, SET-10, and SET-20 and the PK activity was observed, see Fig. 7B-C. TEPP46, a known PK activator, was used as a reference compound.

Treating both HepG2 WT and HepG2 KO protein lysate with 10 µM SET-10 resulted in an inhibitory effect on the PK activity, see Fig. 7B. Treatment of the protein lysates with SET-10 resulted in a 29.4 % reduction in PK activity in HepG2 KO protein lysate and a 28.7 % reduction in PK activity in HepG2 WT protein lysate.

HepG2 WT and HepG2 KO cells were treated with 10 µM of SET-08, SET-10, SET-20 and TEPP46 (as reference compound) for 4 h. Treating the cells with SET-08, SET-10, and SET-20 did not affect the PK activity compared to the control, see Fig. 7C. Treatment of the cells with TEPP46 resulted in an increased activity of the HepG2 WT and HepG2 KO which was expected as it is a known PK activator, see Fig. 7C. The fact that treatment with the target compounds exhibited an inhibitory effect on the PK activity in protein lysates but did not result in any effect in cells, indicates that the target compounds of batch 1 do not enter the cells.

### Batch 2

The effect of treatment of protein lysate and cells (HepG2 WT and HepG2 KO) with SET-02, SET-09, SET-13, SET-15, SET-16, SET-18 and SET-21 on PK activity was evaluated. Urolithin C was evaluated as a reference compound. Treating HepG2 WT and HepG2 KO protein lysate with 10 µM SET-9, SET-13, and SET-15 resulted in an inhibitory effect on PK activity, see Fig. 8A. The inhibitory effect of treatment with the target compounds was SET-09 - 70.2 % (HepG2 KO) and 42.6 % (HepG2 WT), SET-13 - 70.2% (HepG2 KO) and 15.8% (HepG2 WT), SET-15 - 59.1 % (HepG2 KO) and 25.4 % (HepG2 WT).

HepG2 KO and HepG2 WT cells were treated with the target compounds at a concentration of 10 µM for 4 h and PK activity was measured, see Fig. 8B. Treatment of the cells with SET-02 and SET-18 resulted in a small inhibitory effect on PK activity. Treatment with SET-02 inhibited PK activity with 8.8 % (HepG2 KO) and 6.3 % (HepG2 WT) and treatment with SET-18 inhibited PK activity with 7.5 % (HepG2 KO) and 6.2 % (HepG2 WT), all four were statistical significantly.

A DNL steatosis model was treated with 10 µM of the target compounds for 1 week. After which, TAG content, cell viability (MTT assay), and DNL steatosis protein expression were measured, see Fig. 8C. TAG content was further normalized by cell viability and presented as TAG/MTT ratio.

All the evaluated compounds exhibited close to no effect on the cell viability in the DNL steatosis model, see Fig. 8C. SET-09 (21.3 % reduction), SET-13 (21.9 % reduction), and SET-15 (26.8 % reduction) showed a reduction in TAG/MTT ratio compared to the control (DMSO).

### Batch 3

The effect of treatment of protein lysate and cells with 10 µM of SET-25, SET-26, SET-27, SET-27A, SET-35A, SET-43, SET-43A, SET-57, SET-58, SET-59, SET-60, SET-61, SET-62, and SET-80A on PK activity was evaluated. Urolithin C was evaluated as a reference compound. Several of the tested compounds reduced the PK activity in protein lysates, see table 1 and Fig. 9A. Treatment with SET-62 resulted in the highest inhibition of both HepG2 KO and HepG2 WT protein lysate.

Table 1 show the reduction in PK activity of HepG2 KO and HepG2 WT protein lysate treated with target compounds compared to the control.

| Target compound | HepG2 KO (%) | HepG2 WT (%) |
|---|---|---|
| SET-62 | 57.0 | 59.9 |
| SET-57 | 35.2 | 10.7 |
| SET-58 | 32.4 | 33.8 |
| SET-60 | 17.2 | - |
| SET-59 | 11.1 | 13.3 |
| SET-61 | 8.8 | 11.7 |
| SET-26 | 8.0 | - |
| SET-35A | 4.4 | - |
| SET-27A | 4.3 | - |

Furthermore, the PK activity of treated HepG2 KO and HepG2 WT cells was evaluated, see Fig. 9B. The cells were treated with 20 µM of target compounds for 4 h after which PK activity was measured. SET-62 showed a decrease in PK activity of HepG2 KO cell with 32.8 %. Treatment of HepG2 WT cells with the target compounds did not result in any inhibition of PK activity, but rather a slight increase in PK activity when treated with SET-62 (6 %).

SET-62, SET-57, SET-58, SET-60, SET-59, and SET-61 were chosen to proceed with for the treatment of the DNL steatosis model. Urolithin C was used as a reference. The DNL steatosis model was treated with 10 µM of target compounds for 1 week and the TAG content, cell viability and TAG/MTT ratio were determined, see Fig. 9C. Treatment with several of the target compounds, such as SET-62, SET-57, SET-58, SET-59 and SET-60, resulted in reduced TAG content while only treatment with SET-62 resulted in decreased cell viability. Treatment with SET-62 resulted in the largest decrease of TAG content and cell viability and the TAG/MTT ratio was 92.9 %. Treatment with SET-57 and SET-60 also resulted in a reduction in TAG/MTT ratio, 15.6 % for SET-57 and 16.3 % for SET-60.

As 10 µM of the target compound SET-62 exhibited a strong toxicity as evident by the low cell viability, see Fig. 9C, lower doses were further evaluated. A DNL steatosis model was hence treated with 5 µM, 2.5 µM, 1.25 µM, and 0.625 µM of SET-62 and TAG content and cell viability were measured, see Fig. 9D.

Treatment with 5 µM of SET-62 reduced the TAG/MTT ratio to 49.4 % and treatment with 0.625 µM reduced TAG/MTT to 15.0 %. In fact, the lower doses of SET-62 did not exhibit any toxicity as evident by the high cell viability, see Fig. 9D.

Treatment with SET-62 also resulted in decreased PKL and fatty acid synthase (FASN) protein expression level.

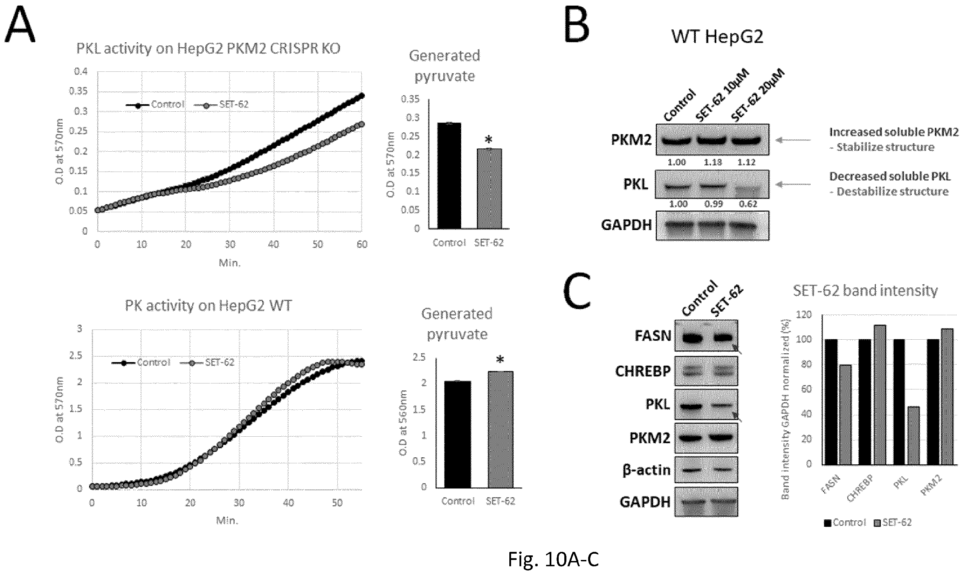

Furthermore, Cesta was performed in order to elucidate the opposite effect treatment with SET-62 had on HepG2 KO cells (inhibition) and HepG2 WT cells (activation), see Fig. 10A. HepG2 WT cells were treated with two different concentrations, 10 and 20 µM, of SET-62 for 2 h and the solubility of PKL and PKM2 was analysed. SET-62 treatment increased the solubility of PKM2 with 12-18 % while it decreased the solubility of PKL with ~38 %. These results indicate that SET-62 stabilizes the PKM2 structure as a chaperon protein (Hsps) but destabilize the PKL structure, see Fig. 10B. This may also explain why the PKL expression level was decreased in the DNL steatosis model, see Fig. 10C. Destabilized PKL was cellularly degraded and thus reduced the expression level of FASN as well.

### Batch 4

A new batch of target compound was assessed in regard to PK activity on HepG2 KO and HepG2 WT protein lysate, see Fig. 11A. Urolithin C was used as a reference compound and SET-62 was tested together with batch 4 due to it high efficiency. Treatment with several of the target compounds reduced the PK activity in both HepG2 KO and HepG2 WT protein lysate, with SET-68D showing similar or slightly higher inhibition than SET-62, followed by SET-69D, SET-69B, SET68B, and SET-66B.

The inhibitory effect of these compounds (together with SET-62) on PK activity was further tested on HepG2 KO and HepG2 WT cells, see Fig. 11B. Treatment of the cells was performed with 20 µM of the target compounds for 4 h.

Only treatment with SET-62 resulted in an effect in the PK activity while the rest of the tested target compound did not seem to affect the PK activity in the cells. Treatment with SET-62 resulted in a decrease (24.4 %) in PK activity in HepG2 KO cells and a slight increase (9.0%) in PK activity in HepG2 WT cells as observed before.

DNL steatosis model was further treated for 1 week with 5 µM of SET-62, SET-68D, SET-69D, SET-69B, SET-68B, and SET-66B, respectively, and TAG content, cell viability and TAG/MTT ratio were assessed, see Fig. 11C. Treating the DNL steatosis model with 5 µM SET-62 decreased the TAG/MTT ratio to 61.3% while treatment with SET-66B slightly increased (6.0%) the TAG/MTT ratio. Furthermore, the cell viability after treatment with the target compounds of batch 4 was slightly reduced, however, treatment with SET-62 increased the cell viability.

The expression of DNL involved steatosis proteins after treatment with target compounds was evaluated, see Fig. 11C. It was observed that treatment with 5 µM of SET-62 decreased PKL and FASN expression. Furthermore, a slight increase in the expression of PKM2 was observed after treatment with SET-62 which correspond with previous results.

Treatment with SET-68D resulted in the highest inhibitory effect on PK activity when tested on HepG2 WT protein lysate indicating excellent PK inhibition. Although, the same results were not obtained when tested on cells (indicating that the compound does not enter the cells), it is believed that if provided in a formulation that facilitates cell entry, SET-68D may be used as a medicament for NAFLD.

### Batch 5

A final batch of target compound was assessed in regard to PK activity on HepG2 KO and HepG2 WT protein lysate, see Fig. 12A. Urolithin C was used as a reference compound and SET-62 was tested together with batch 5 due to it high efficiency. Treatment with several target compounds exhibited a reduction in PK activity of both HepG2 KO and HepG2 WT protein lysate, see Fig. 12A. Treatment with SET-62 still resulted in the highest inhibitory effect of all tested target compounds.

The compounds whose treatment exhibited to the most inhibitory effect on PK activity in HepG2 KO and HepG2 WT protein lysates, respectively, were chosen to be tested on cells, see Fig. 12B. SET-77D, SET-75D, SET-74B, SET-78D, SET-79B, SET-75B, and SET-77B were chosen together with SET-62 and the effect of their treatment was evaluated on HepG2 KO cells. SET-78D, SET-74B, SET-75B, SET-79B, and SET-75D were chosen together with SET-62 and the effect of their treatment was evaluated on HepG2 WT cells. The cells were treated with 20 µM of the target compounds for 4 h. Only treatment with SET-62 resulted in a change in PK activity in cells. As shown before, treatment with SET-62 decreased the PK activity in HepG2 KO cells and slightly increased the PK activity in HepG2 WT cells.

These experiments show that treatment with SET-62 reduces the PKL activity in both protein lysates and in HepG2 cells. Furthermore, it was shown that treatment with SET-62 reduced the TAG content in a DNL steatosis model making it suitable for NAFLD and NASH treatment.

### REFERENCES

[1] Marjot, T.; Moolla, A.; Cobbold, J.F.; Hodson, L.; Tomlinson, J.W. Nonalcoholic Fatty Liver Disease in Adults: Current Concepts in Etiology, Outcomes, and Management. Endocr. Rev. 2020, 41, 66-117, doi:10.1210/endrev/bnz009.
[2] Lee, S.; Zhang, C.; Liu, Z.; Klevstig, M.; Mukhopadhyay, B.; Bergentall, M.; Cinar, R.; Stahlman, M.; Sikanic, N.; Park, J.K.; et al. Network analyses identify liver-specific targets for treating liver diseases. Mol. Syst. Biol.2017, 13, 938, doi:https://doi.org/10.15252/msb.20177703.
[3]Younossi, Z.M.; Koenig, A.B.; Abdelatif, D.; Fazel, Y.; Henry, L.; Wymer, M. Global epidemiology of nonalcoholic fatty liver disease-Meta-analytic assessment of prevalence, incidence, and outcomes. Hepatology 2016, 64, 73-84, doi:https://doi.org/10.1002/hep.28431.
[4]Huang, D.Q.; El-Serag, H.B.; Loomba, R. Global epidemiology of NAFLD-related HCC: trends, predictions, risk factors and prevention. Nat. Rev. Gastroenterol. Hepatol. 2021, 18, 223-238, doi:10.1038/s41575-020-00381-6.
[5]Chalasani, N.; Younossi, Z.; Lavine, J.E.; Diehl, A.M.; Brunt, E.M.; Cusi, K.; Charlton, M.; Sanyal, A.J. The Diagnosis and Management of Non-alcoholic Fatty Liver Disease: Practice Guideline by the American Gastroenterological Association, American Association for the Study of Liver Diseases, and American College of Gastroenterology. Gastroenterology 2012, 142, 1592-1609, doi:https://doi.org/10.1053/j.gastro.2012.04.001.
[6] Chella Krishnan, K.; Kurt, Z.; Barrere-Cain, R.; Sabir, S.; Das, A.; Floyd, R.; Vergnes, L.; Zhao, Y.; Che, N.; Charugundla, S.; et al. Integration of Multi-omics Data from Mouse Diversity Panel Highlights Mitochondrial Dysfunction in Non-alcoholic Fatty Liver Disease. Cell Syst. 2018, 6, 103-115.6107, doi:https://doi.org/10.1016/j.cels.2017.12.006.
[7]Liu, Z.; Zhang, C.; Lee, S.; Kim, W.; Klevstig, M.; Harzandi, A.M.; Sikanic, N.; Arif, M.; Stahlman, M.; Nielsen, J.; et al. Pyruvate kinase L/R is a regulator of lipid metabolism and mitochondrial function. Metab. Eng. 2019, 52, 263-272, doi:https://doi.org/10.1016/j.ymben.2019.01.001.
[8] Muirhead, H. Isoenzymes of pyruvate kinase. Biochem. Soc. Trans. 1990, 18, 193-196, doi:10.1042/bsto180193.
[9]Christofk, H.R.; Vander Heiden, M.G.; Harris, M.H.; Ramanathan, A.; Gerszten, R.E.; Wei, R.; Fleming, M.D.; Schreiber, S.L.; Cantley, L.C. The M2 splice isoform of pyruvate kinase is important for cancer metabolism and tumour growth. Nature 2008, 452, 230-233, doi:10.1038/nature06734.
[10]Goldberg, M.S.; Sharp, P.A. Pyruvate kinase M2-specific siRNA induces apoptosis and tumor regression. J- Exp. Med. 2012, 209, 217-224, doi:10.1084/jem.20111487.
[11] Yamada, K.; Noguchi, T. Nutrient and hormonal regulation of pyruvate kinase gene expression. Biochem. J. 1998, 337, 1-11, doi:10.1042/bj3370001.
[12] Nain-Perez, A.; Foller Füchtbauer, A.; Haversen, L.; Lulla, A.; Gao, C.; Matic, J.; Monjas, L.; Rodriguez, A.; Brear, P.; Kim, W.; et al. Anthraquinone derivatives as ADP-competitive inhibitors of liver pyruvate kinase. Eur. J. Med. Chem. 2022, 234, 114270, doi:https://doi.org/10.1016/j.ejmech.2022.114270.
[13] Battisti, U.M.; Gao, C.; Nilsson, O.; Akladios, F.; Lulla, A.; Bogucka, A.; Nain-Perez, A.; Haversen, L.; Kim, W.; Boren, J.; et al. Serendipitous Identification of a Covalent Activator of Liver Pyruvate Kinase. ChemBioChem 2023, 24, e202200339, doi:https://doi.org/10.1002/cbic.202200339.
[14] Battisti, U.M.; Gao, C.; Akladios, F.; Kim, W.; Yang, H.; Bayram, C.; Bolat, I.; Kiliclioglu, M.; Yuksel, N.; Tozlu, O.O.; et al. Ellagic Acid and Its Metabolites as Potent and Selective Allosteric Inhibitors of Liver Pyruvate Kinase. Nutrients 2023, 15, doi:10.3390/nu15030577.
[15] "Molecular Operating Environment (MOE), 2019.01; Chemical Computing Group ULC, 1010 Sherbooke St. West, Suite #910, Montreal, QC, Canada, H3A 2R7, 2019." 2019.
[16] Angell RM, Atkinson FL, Brown MJ, Chuang TT, Christopher JA, Cichy-Knight M, Dunn AK, Hightower KE, Malkakorpi S, Musgrave JR, Neu M. N-(3-Cyano-4, 5, 6, 7-tetrahydro-1-benzothien-2-yl) amides as potent, selective, inhibitors of JNK2 and JNK3. Bioorganic & medicinal chemistry letters. 2007 Mar 1;17(5):1296-301.
[17] Halgren, Thomas A. 1996. "Merck Molecular Force Field. I. Basis, Form, Scope, Parameterization, and Performance of MMFF94." Journal of Computational Chemistry 17 (5-6): 490-519.
[18]Zhang, C. et al. Discovery of therapeutic agents targeting PKLR for NAFLD using drug repositioning. eBioMedicine 83, 104214 (2022).
[19] Qi, W. et al. Pyruvate kinase M2 activation may protect against the progression of diabetic glomerular pathology and mitochondrial dysfunction. Nat Med 23, 753-762 (2017).

## Claims

1. A compound according to formula (I) 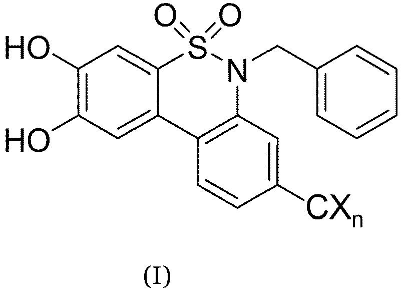 or a pharmaceutically acceptable salt or prodrug thereof wherein X is a halogen and n=1-3.

2. The compound according to claim 1, wherein X is fluoride.

3. The compound according to claim 1 or 2, wherein n is 2 or 3, preferably 3.

4. The compound according to claim 3, having the formula (II)

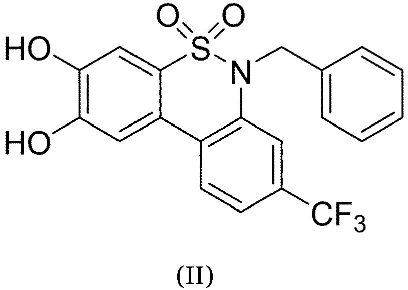

5. A pharmaceutical composition comprising a compound according to any one of the preceding claims.

6. A compound or a pharmaceutical composition according to any one of the preceding claims for use in a method of treatment of fatty liver disease or hepatocellular carcinoma (HCC).

7. The compound or pharmaceutical composition for use according to claim 6, wherein the method of treatment comprises oral administration of the compound.

8. The compound or pharmaceutical composition for use according to claim 6 or 7, wherein said fatty liver disease is non-alcoholic fatty liver disease (NAFLD) and said NAFLD optionally has progressed to non-alcoholic steatohepatitis (NASH).
